# EUROPEAN PATENT SPECIFICATION

(11) **EP 2 197 468 B1**
(45) Date of publication and mention of the grant of the patent: **29.07.2015**
(21) Application number: 08842661.4
(22) Date of filing: 24.10.2008
(51) Int. Cl.: A61K 38/16, C07K 14/00, A61K 47/48, A61K 51/10, A61K 38/18, A61K 38/19, A61K 38/20, A61K 38/21, A61K 51/08

(54) **PEGYLATION BY THE DOCK AND LOCK (DNL) TECHNIQUE**
PEGYLIERUNG MITHILFE DES DOCK-AND-LOCK-VERFAHRENS
PEGYLATION PAR LA TECHNIQUE D'ACCOSTAGE ET VERROUILLAGE (DNL)

(30) Priority: 26.10.2007 US 925408
(43) Date of publication of application: 23.06.2010
(73) Proprietor: IBC Pharmaceuticals, Inc., Morris Plains, NJ 07950 (US)
(72) Inventor: MCBRIDE, William, Morris Plains NJ 07950 (US); CHANG, Chien-hsing, Morris Plains NJ 07950 (US); ROSSI, Edmund, Morris Plains NJ 07950 (US); GOLDENBERG, David, Morris Plains NJ 07950 (US)
(74) Representative: Bohmann, Armin K.
(86) International application number: PCT/US2008/081085
(87) International publication number: WO 2009/055653

(56) References cited:
- WO-A2-2007/075270
- US-A1- 2006 228 300
- US-A1- 2006 228 357
- US-A1- 2007 086 942
- US-B2- 6 831 158
- CHANG C-H ET AL: "The Dock and Lock Method: A Novel Platform Technology for Building Multivalent, Multifunctional Structures of Defined Composition with Retain Bioactivity", CLINICAL CANCER RESEARCH, THE AMERICAN ASSOCIATION FOR CANCER RESEARCH, US, vol. 13, no. 18 SUPPL, 15 September 2007 (2007-09-15), pages 5586s-5591s, XP008123543, ISSN: 1078-0432, DOI: 10.1158/1078-0432.CCR-07-1217

## Description

The present invention is related to a PEGylated complex and a method for PEGylating an effector moiety.

### BACKGROUND

The efficacy of a therapeutic agent may be enhanced by improving its bioavailability via several means, one of which is PEGylation, a process of chemically linking polyethylene glycol (PEG) to the therapeutic agent of interest, with the resulting conjugate exhibiting an increased serum half-life. Additional advantages of the PEGylated products may also include lower immunogenicity, decreased dosing frequency, increased solubility, enhanced stability, and reduced renal clearance. Because the most common reactive sites on proteins (including peptides) for attaching PEG are the ε amino groups of lysine and the α amino group of the N-terminal residue, early methods of PEGylation resulted in modification of multiple sites, yielding not only monoPEGylated conjugates consisting of mixtures of positional isomers, such as PEGINTRON™ (Grace et al., J. Biol. Chem. 2005; 280:6327) and PEGASYS® (Dhalluin et al., Bioconjugate Chem. 2005; 16:504), but also adducts comprising more than one PEG chain. Site-specific attachment of a single PEG to the α amino group of the N-terminal residue was reported to be the predominant product upon reacting PEG-aldehyde (PEG-ALD) at low pH with IFN-β1b (Basu et al., Bioconjugate Chem. 2006;17:618) or IFN-β1a (Pepinsky et al., J. Pharmacol. Exp. Ther. 2001;297:1059). Similar strategies were applied to prepare N-terminally linked PEG to G-CSF (Kinstler et al., Pharm. Res. 1996;13:996) or type I soluble tumor necrosis factor receptor (Kerwin et al., Protein Sci. 2002;11:1825). More recently, a solid-phase process for PEGylation of the N-terminus of recombinant interferon alpha-2a was reported (Lee et al., Bioconjug. Chem. Oct. 18, 2007, epub).

Site-directed PEGylation of a free cysteine residue introduced into a target protein has also been achieved with PEG-maleimide (PEG-MAL) for several recombinant constructs including IL-2 (Goodson and Katre, Biotechnology. 1990:8:343); IFN-α2 (Rosendahl et al., Bioconjugate Chem. 2005;16:200); GM-CSF (Doherty et al., Bioconjugate Chem. 2005;16:1291); scFv (Yang et al., Protein Eng. 2003;16:761), and miniantibodies (Kubetzko et al., J. Biol. Chem; 2006;201:35186). A popular approach for improving the therapeutic efficacy of an enzyme has been to prepare conjugates containing multiple PEG of small size, as known for methioninase (Yang et al., Cancer Res. 2004;64:6673); L-methione γ-lyase (Takakura et al., Cancer Res. 2006:66:2807): arginine deaminase (Wang et al., Bioconjugate Chem. 2006;17:1447); adenosine deaminase (Davis et al., Clin. Exp. Immunol. 1981;46:649); L-asparaginase (Bendich et al., Clin. Exp. Immunol. 1982;48:273); and liver catalase (Abuchowski et al., J. Biol. Chem. 1977;252:3582).

PEGylations of bovine serum albumin (Abuchowski et al., J. Biol. Chem. 1977;252:3578); hemoglobin (Manjula et al., Bioconjugate Chem. 2003;14:464); visomant (Mosharraf et al., Int. J. Pharm. 2007;336:215); small molecules such as inhibitors of integrin ex.4β1 (Pepinsky et al., J. Pharmacol. Exp. Ther. 2005;312:742); lymphoma-targeting peptides (DeNardo et al., Clin. Cancer. Res. 2003;9(Suppl.):3854s); anti-VEGF aptamer (Bunka and Stockley, Nat. Rev. Microbiol. 2006;4:588) and oligodeoxynucleotides (Fisher et al., Drug Metab. Dispos. 2004;32:983) have also been described. However, there exists a need for a general method of PEGylation that would produce exclusively a monoPEGylated conjugate composed of a single PEG linked site-specifically to a predetermined location of the candidate agent and retains the bioactivity of the unmodified counterpart.

WO 2007/075270 A2 is related to methods and compositions for stably tethered structures of defined compositions which may have multiple functionalities and/or binding specificities. Among others, hexameric stably tethered structures comprising one or more IgG antibody fragments are disclosed, whereby such structures may be monospecific or bispecific.

Chang C-H et al. (Clinical Cancer Research, vol. 13, no. 18 suppl, 15 September 2007, pages 5586s-5591s) report on the Dock and Lock method as a novel platform technology for building multivalent, multifunctional structures of defined composition with retained bioactivity.

### SUMMARY OF THE INVENTION

The problem underlying the present invention is solved by the subject matter of the attached independent claims. Preferred embodiments may be taken from the attached dependent claims.

More specifically, the problem underlying the present invention is solved in a first aspect by a PEGylated complex comprising:
a) an effector moiety attached to a dimerization and docking domain (DDD) sequence of protein kinase A (PKA) RIIα, wherein the effector moiety is an interferon (IFN)-α2b ; and
b) a PEG moiety attached to an anchor domain (AD) sequence of an A-kinase anchoring protein (AKAP);
wherein two DDD sequences bind to one AD sequence to form a PEGylated
complex.

In an embodiment of the first aspect, the complex comprises further disulfide bonds between the DDD and AD sequences.

In an embodiment of the first aspect, the DDD sequence comprises the 44 N-terminal amino acids of PKA RIIa.

In an embodiment of the first aspect, the DDD sequence comprises SEQ ID NO:2.

In an embodiment of the first aspect, the PEG moiety is capped at one end with a methoxy group.

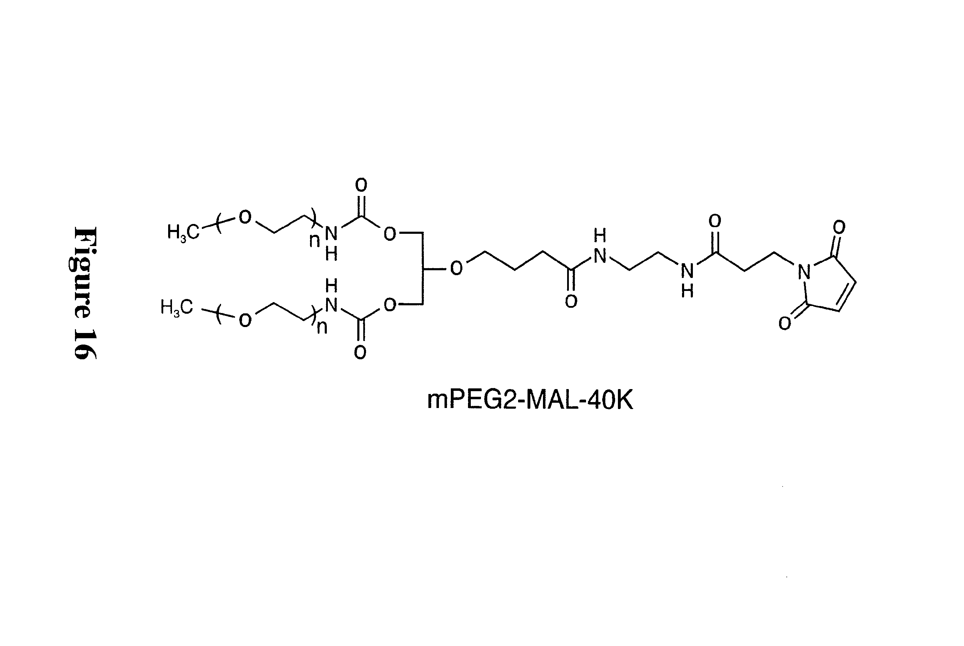

In an embodiment of the first aspect, the PEG moiety attached to an AD sequence comprises IMP350, IMP360, IMP362, IMP413 or IMP457,
wherein
IMP350 is CGQIEYLAKQIVDNAIQQAGC(SS-tbu)-NH₂ (SEQ ID NO:1),
IMP360 is CGQIEYLAKQIVDNAIQQAGC(SS-tbu)G-EDANS (SEQ ID NO:1),
IMP362 is PEG₂₀- CGQIEYLAKQIVDNAIQQAGC(SS-tbu)G-EDANS,
IMP413 is PEG₃₀- CGQIEYLAKQIVDNAIQQAGC(SS-tbu)G-EDANS, and
IMP457 is the reaction product of Ac-C-PEG₃-C(S-*t*Bu)GQIEYLAKQIVDNAIQQAGC(S-*t*Bu)G-NH₂ and mPEG2-MAL-40K of Fig. 16 reacted as described in Example 1.

In an embodiment of the first aspect, the clearance rate of the PEGylated complex from serum is at least an order of magnitude slower than the clearance rate of the unPEGylated effector moiety.

More specifically, the problem underlying the present invention is solved in a second aspect by a PEGylated complex comprising:
a) an effector moiety attached to an anchor domain (AD) sequence of an A-kinase anchoring protein (AKAP), wherein the effector moiety is an interferon (IFN)-α2b; and
b) a PEG moiety attached to a dimerization and docking domain (DDD) sequence of protein kinase A (PKA) RIIα;
wherein two DDD sequences bind to one AD sequence to form a PEGylated complex.

In an embodiment of the second aspect, the clearance rate of the PEGylated complex from serum is at least an order of magnitude slower than the clearance rate of the unPEGylated effector moiety.

More specifically, the problem underlying the present invention is solved in a third aspect by a method of PEGylating an effector moiety comprising:
a) attaching an effector moiety to a dimerization and docking domain (DDD) sequence of protein kinase A (PKA) RIIa, wherein the effector moiety is an interferon (IFN)-α2b ;
b) attaching a PEG moiety to an anchor domain (AD) sequence of an A-kinase anchoring protein (AKAP); and
c) allowing the DDD sequence to bind to the AD sequence to form a PEGylated complex comprising two effector moiety-DDD sequences and one PEG-AD sequence.

In an embodiment of the third aspect, the clearance rate of the PEGylated complex from serum is at least an order of magnitude slower than the clearance rate of the unPEGylated effector moiety.

More specifically, the problem underlying the present invention is solved in a fourth aspect by a method of PEGylating an effector moiety comprising:
a) attaching a effector moiety to an anchor domain (AD) sequence of an A-kinase anchoring protein (AKAP), wherein the effector moiety is an interferon (IFN)-α2b ;
b) attaching a PEG moiety to a dimerization and docking domain (DDD) sequence of protein kinase A (PKA) RIIα; and
c) allowing the DDD sequence to bind to the AD sequence to form a PEGylated complex comprising two effector moiety-DDD sequences and one PEG-AD sequence.

The present invention discloses methods and compositions for producing PEGylated compounds with selected numbers of attached PEG residues that are attached at selected locations of a candidate agent as defined in the claims. In preferred embodiments, the agents are monoPEGylated. In more preferred embodiments, the target to be PEGylated may be attached to a DDD (dimerization and docking domain) sequence and a PEG moiety may be attached to an AD (anchor domain) sequence as described in more detail below. Dimers of the DDD sequence bind with high affinity to monomers of the AD sequence, resulting in formation of a monoPEGylated effector moiety dimer. The stoichiometry of binding and location of the PEG residue are determined by the specificity of the DDD/AD interaction.

In more preferred embodiments, the monoPEGylated complex may be covalently stabilized by introduction of cysteine residues at appropriate locations in the DDD and AD sequences, to form disulfide bonds that stabilize the complex. In other embodiments, the PEG reagents may be capped at one end with a linear or branched methoxy group (m-PEG).

In other preferred embodiments, the PEGylated complex made by the DNL method shows a rate of clearance from serum that is at least an order of magnitude slower than the unPEGylated effector moiety. In certain alternative embodiments, the PEGylated complex may be alternatively constructed with the PEG moiety attached to the DDD sequence and the effector moiety attached to the AD sequence, resulting in a stoichiometry of 2 PEG to 1 effector moiety per complex.

The skilled artisan will realize that virtually any physiologically or therapeutically active agent to be administered *in vivo* may be stabilized by PEGylation, including but not limited to enzymes, cytokines, chemokines, growth factors, peptides, apatamers, hemoglobins, antibodies and fragments thereof. Exemplary agents include MIF, HMGB-1 (high mobility group box protein 1), TNF-α, IL-1, IL-2, IL-3, IL-4, IL-5, IL-6, IL-7, IL-8, IL-9, IL-10, IL-11, IL-12, IL-13, IL-15, IL-16, IL-17, IL-18, IL-19, IL-23, IL-24, CCL19, CCL21, IL-8, MCP-1, RANTES, MIP-1A, MIP-1B, ENA-78, MCP-1, IP-10, Gyro-β, Eotaxin, interferon-α, -β, -λ, G-CSF, GM-CSF, SCF, PDGF, MSF, Flt-3 ligand, erythropoietin, thrombopoietin, hGH, CNTF, leptin, oncostatin M, VEGF, EGF, FGF, P1GF, insulin, hGH, calcitonin, Factor VIII, IGF, somatostatin, tissue plasminogen activator, and LIF.

The monoPEGylated complexes, are suitable for use in a wide variety of therapeutic and diagnostic applications. Methods of use of monoPEGylated complexes may include detection, diagnosis and/or treatment of a disease or other medical condition. Such conditions may include, but are not limited to, cancer, hyperplasia, diabetes, diabetic retinopathy, macular degeneration, inflammatory bowel disease, Crohn's disease, ulcerative colitis, rheumatoid arthritis, sarcoidosis, asthma, edema, pulmonary hypertension, psoriasis, corneal graft rejection, neovascular glaucoma, Osler-Webber Syndrome, myocardial angiogenesis, plaque neovascularization, restenosis, neointima formation after vascular trauma, telangiectasia, hemophiliac joints, angiofibroma, fibrosis associated with chronic inflammation, lung fibrosis, deep venous thrombosis or wound granulation.

In particular embodiments, PEGylated complexes and the disclosed compositions may be of use to treat autoimmune disease, such as acute idiopathic thrombocytopenic purpura, chronic idiopathic thrombocytopenic purpura, dermatomyositis, Sydenham's chorea, myasthenia gravis, systemic lupus erythematosus, lupus nephritis, rheumatic fever, polyglandular syndromes, bullous pemphigoid, juvenile diabetes mellitus, Henoch-Schonlein purpura, post-streptococcal nephritis, erythema nodosurn, Takayasu's arteritis, Addison's disease, rheumatoid arthritis, multiple sclerosis, sarcoidosis, ulcerative colitis, erythema multiforme, IgA nephropathy, polyarteritis nodosa, ankylosing spondylitis, Goodpasture's syndrome, thromboangitis obliterans, Sjogren's syndrome, primary biliary cirrhosis, Hashimoto's thyroiditis, thyrotoxicosis, scleroderma, chronic active hepatitis, polymyositis/dermatomyositis, polychondritis, pemphigus vulgaris, Wegener's granulomatosis, membranous nephropathy, amyotrophic lateral sclerosis, tabes dorsalis, giant cell arteritis/polymyalgia, pernicious anemia, rapidly progressive glomerulonephritis, psoriasis or fibrosing alveolitis.

It is anticipated that any type of tumor and any type of tumor antigen may be targeted. Exemplary types of tumors that may be targeted include acute lymphoblastic leukemia, acute myelogenous leukemia, biliary cancer, breast cancer, cervical cancer, chronic lymphocytic leukemia, chronic myelogenous leukemia, colorectal cancer, endometrial cancer, esophageal, gastric, head and neck cancer, Hodgkin's lymphoma, lung cancer, medullary thyroid cancer, non-Hodgkin's lymphoma, multiple myeloma, renal cancer, ovarian cancer, pancreatic cancer, glioma, melanoma, liver cancer, prostate cancer, and urinary bladder cancer.

### BRIEF DESCRIPTION OF THE DRAWINGS

**FIG. 1****.** Cartoon illustration of the DNL method. Triangles depict component A, which forms a homodimer (**a₂**) mediated by the dimerization and docking domain (**DDD**). The location of free thiol groups (**SH**) of the engineered cysteine residues is indicated. Octagons depict component **B** containing an anchor domain (**AD**) peptide. The DNL reaction results in the generation of a covalent trimeric structure via binding of DDD and AD peptides and subsequent formation of disulfide bridges.
**FIG. 2****.** Cartoon drawing of IMP362. A 20 kDa PEG (starburst), AD2 peptide (helix), EDANS fluorescent tag (oval) and the positions of free sulfhydryl groups (SH) are indicated.
**FIG. 3****.** Cartoon drawing of IMP413. A 30 kDa PEG (starburst), AD2 peptide (helix), EDANS fluorescent tag (oval) and the positions of free sulfhydryl groups (SH) are indicated.
**FIG. 4****.** Analysis of roller bottle production and purification by anti-IFNa immunoblot and ELISA. Samples were diluted as indicated and 5 µl were subjected to reducing SDS-PAGE and immunoblot analysis with polyclonal anti-IFNα. The dilution, total volume and fraction analyzed of the total volume (f) for each sample is given. The amount of protein in each band was estimated from standards and divided by the total volume to give the total protein estimate. Total protein measurements determined by ELISA are also given.
**FIG. 5****.** Cartoon drawing of α2b-362. IFNα2b groups (pentagons), 20 kDa PEG (starburst), AD2 and DDD2 peptides (helices) and EDANS fluorescent tag (oval) are indicted.
**FIG. 6****.** Cartoon drawing of α2b-413. IFNα2b groups (pentagons), 30 kDa PEG (starburst), AD2 and DDD2 peptides (helices) and EDANS fluorescent tag (oval) are indicted.
**FIG. 7****.** Dose-response curves showing in vitro growth inhibition of Burkitt's lymphoma (Daudi) cells after 4-days in culture in the presence of either rhIFN-α2b standard, IFN-α2b-DDD2 or α2b-362. MTS dye was added to the plates, which were incubated for 3 h before measuring the OD₄₉₀. The % of the signal obtained from untreated cells was plotted vs. the log of the molar concentration. The 50% effective concentration (EC₅₀) values were obtained by sigmoidal fit non-linear regression using Graph Pad Prism software.
**FIG. 8****.** Evaluation of the phannacokinetic properties of IFNα constructs. Each reagent (test and control) was administered to Swiss-Webster mice at equimolar protein doses as a single bolus i.v. injection of 3 µg for rhuIFN-α2a, 5 µg for PEGINTRON™, 11 µg for α2b-362, and 13 µg for α2b-413. Serum samples were isolated at the times indicated and the serum concentrations of IFN-α were determined by ELISA. The pM concentration was plotted vs. hours post injection. Data represents the mean value from two mice.
**FIG. 9****.** Evaluation of the therapeutic efficacy of IFNα constructs in mice bearing Burkitt's lymphoma (Daudi). Eight-week-old female SCID mice were injected i.v. with 1.5 x 10⁷ Daudi cells. Groups of 5 mice were administered PEGINTRON™ , α2b-362 and α2b-413 at doses of 3,500, 7,000 or 14,000 Units once per week for 4 weeks. Therapy commenced 1 day after the Daudi cells were transplanted. Injection times are indicated with arrows. Survival curves and median survival are shown for each group.

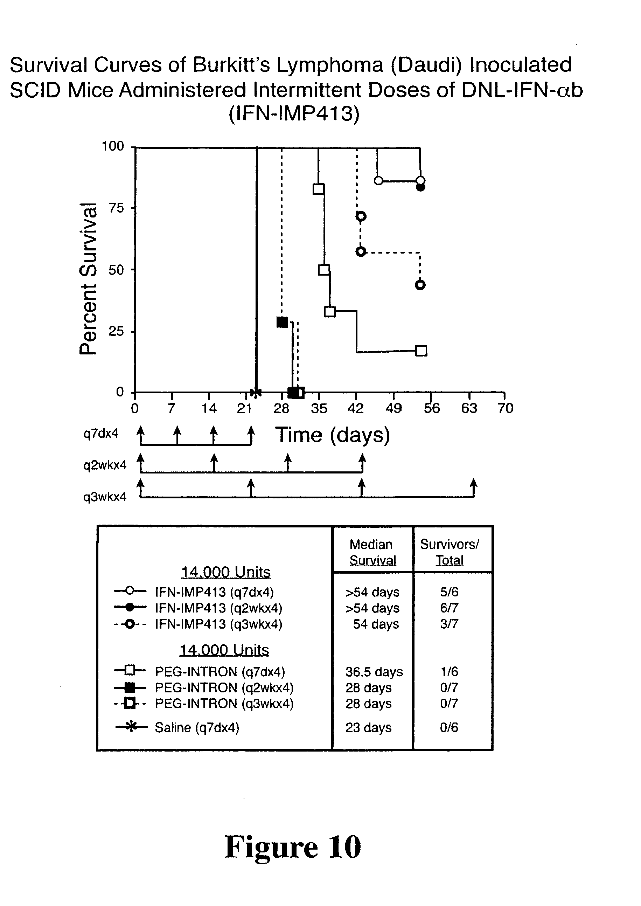

**FIG. 10****.** Evaluation of the dosing schedule for therapy of tumor-bearing mice. Eight-week-old female SCID mice were injected i.v. with 1.5 x 10⁷ Daudi-cells. Groups of 6-7 mice were administered 14,000 IU of either PEGINTRON™ or α2b-413 *via* a s.c. injection. Therapy was commenced 1 day after the Daudi cells were administered to the mice. Groups were dosed once a week (q7dx4), once every other week (q2wkx4) or once every 3 weeks (q3wkx4). Injection times are indicated with arrows. All the mice received 4 injections in total. Survival curves and median survival are shown for each group.
**FIG. 11****.** Cartoon drawing of G-CSF-413. G-CSF groups (pentagons), 30 kDa PEG (starburst), AD2 and DDD2 peptides (helices) and EDANS fluorescent tag (oval) are indicted.
**FIG. 12****.** Cartoon drawings of h679-Fab-DDD2 (A), dimeric EPO-DDD2 (B), which combine to create EPO-679 (C) by the DNL method. The variable and constant domains of h679 Fab (ovals), AD2 and DDD2 helices, EPO groups (pentagons) and free sulfhydryl groups (SH) are indicated.
**FIG. 13**. Stimulation of cell growth by EPO-constructs. EPO-responsive TF1 cells (1 x 10⁴) were cultured for 72 hours in the presence of rhEPO, EPO-DDD2 or EPO-679. The relative viable cell density was determined by MTS assay. The log of the concentration in U/mL is plotted vs. OD₄₉₀.
**FIG. 14****.** Cartoon drawing of EPO-413. EPO groups (pentagons), 30 kDa PEG (starburst), AD2 and DDD2 peptides (helices) and EDANS fluorescent tag (oval) are indicted.
**FIG. 15****.** Structure of IMP-421.
**FIG. 16****.** Structure of mPEG2-MAL-40K.

### Dock and Lock (DNL) method

The DNL method exploits specific protein/protein interactions that occur between the regulatory (R) subunits of cAMP-dependent protein kinase (PKA) and the anchoring domain (AD) of A-kinase anchoring proteins (AKAPs) (Baillie et al., FEBS Letters. 2005; 579: 3264. Wong and Scott, Nat. Rev. Mol. Cell Biol. 2004; 5: 959). PKA, which plays a central role in one of the best studied signal transduction pathways triggered by the binding of the second messenger cAMP to the R subunits, was first isolated from rabbit skeletal muscle in 1968 (Walsh et al., J. Biol. Chem. 1968;243:3763). The structure of the holoenzyme consists of two catalytic subunits held in an inactive form by the R subunits (Taylor, J. Biol. Chem. 1989;264:8443). Isozymes of PKA are found with two types of R subunits (RI and RII), and each type has α and β isoforms (Scott, Pharmacol. Ther. 1991;50:123). The R subunits have been isolated only as stable dimers and the dimerization domain has been shown to consist of the first 44 amino-terminal residues (Newlon et al., Nat. Struct. Biol. 1999;6:222). Binding of cAMP to the R subunits leads to the release of active catalytic subunits for a broad spectrum of serine/threonine kinase activities, which are oriented toward selected substrates through the compartmentalization of PKA via its docking with AKAPs (Scott et al., J. Biol. Chem. 1990;265;21561)

Since the first AKAP, microtubule-associated protein-2, was characterized in 1984 (Lohmann et al., Proc. Natl. Acad. Sci USA. 1984;81:6723), more than 50 AKAPs that localize to various sub-cellular sites, including plasma membrane, actin cytoskeleton, nucleus, mitochondria, and endoplasmic reticulum, have been identified with diverse structures in species ranging from yeast to humans (Wong and Scott, Nat. Rev. Mol. Cell Biol. 2004;5:959). The AD of AKAPs for PKA is an amphipathic helix of 14-18 residues (Carr et al., J. Biol. Chem. 1991;266:14188). The amino acid sequences of the AD are quite varied among individual AKAPs, with the binding affinities reported for RII dimers ranging from 2 to 90 nM (Alto et al., Proc. Natl. Acad. Sci. USA. 2003;100:4445). Interestingly, AKAPs will only bind to dimeric R subunits. For human RIIα, the AD binds to a hydrophobic surface formed by the 23 amino-terminal residues (Colledge and Scott, Trends Cell Biol. 1999; 6:216). Thus, the dimerization domain and AKAP binding domain of human RIIα are both located within the same N-terminal 44 amino acid sequence (Newlon et al., Nat. Struct. Biol. 1999;6:222; Newlon et al., EMBO J. 2001;20:1651), which is termed the DDD herein.

### DDD of Human RIIα and AD of AKAPs as Linker Modules

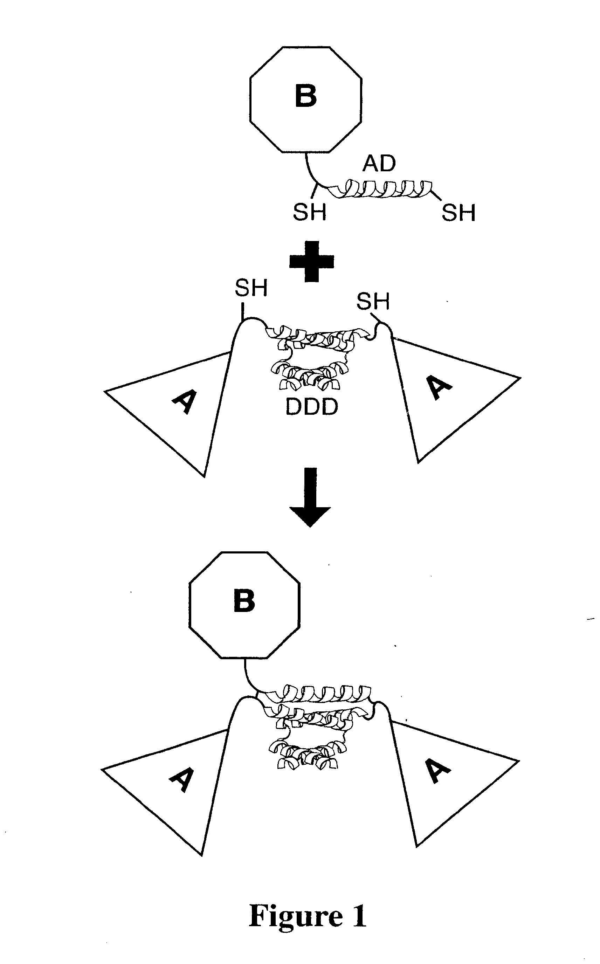

We have developed a platform technology to utilize the DDD of human RIIα, and the AD of a certain amino acid sequence as an excellent pair of linker modules for docking any two entities, referred to hereafter as **A** and **B**, into a noncovalent complex, which could be further locked into a stably tethered structure through the introduction of cysteine residues into both the DDD and AD at strategic positions to facilitate the formation of disulfide bonds, as illustrated in Fig.1. The general methodology of the "dock-and-lock" approach is as follows. Entity **A** is constructed by linking a DDD sequence to a precursor of **A**, resulting in a first component hereafter referred to as **a**. Because the DDD sequence would effect the spontaneous formation of a dimer, **A** would thus be composed of **a₂**. Entity **B** is constructed by linking an AD sequence to a precursor of **B**, resulting in a second component hereafter referred to as **b**. The dimeric motif of DDD contained in **a₂** will create a docking site for binding to the AD sequence contained in **b**, thus facilitating a ready association of **a₂** and **b** to form a binary, trimeric complex composed of **a₂b**. This binding event is made irreversible with a subsequent reaction to covalently secure the two entities via disulfide bridges, which occurs very efficiently based on the principle of effective local concentration because the initial binding interactions should bring the reactive thiol groups placed onto both the DDD and AD into proximity (Chimura et al., Proc. Natl. Acad. Sci. USA. 2001;98:8480) to ligate site-specifically.

By attaching the DDD and AD away from the functional groups of the two precursors, such site-specific ligations are also expected to preserve the original activities of the two precursors. This approach is modular in nature and potentially can be applied to link, site-specifically and covalently, a wide range of substances, including peptides, proteins, nucleic acids, and PEG. The DNL method was disclosed in U.S. provisional patent applications 60/728,292, filed October 20, 2005; 60/751,196, filed December 16, 2005; and 60/782,332, filed March 14, 2006; and U.S. patent applications 11/389,358, filed March 24, 2006; 11/391,584, filed March 28, 2006; 11/478,021, filed June 29, 2006; 11/633,729, filed December 5, 2006 and 11/925,408, filed October 26, 2007.

As illustrated in the Examples below, the effector moiety to be PEGylated is a protein or peptide, which can be linked to a DDD or AD unit to form a fusion protein or peptide. A variety of methods are known for making fusion proteins, including nucleic acid synthesis, hybridization and/or amplification to produce a synthetic double-stranded nucleic acid encoding a fusion protein of interest. Such double-stranded nucleic acids may be inserted into expression vectors for fusion protein production by standard molecular biology techniques (see, e.g. Sambrook et al., Molecular Cloning, A laboratory manual, 2nd Ed, 1989). In such preferred embodiments, the AD and/or DDD moiety may be attached to either the N-terminal or C-terminal end of an effector protein or peptide. However, the skilled artisan will realize that the site of attachment of an AD or DDD moiety to an effector moiety may vary, depending on the chemical nature of the effector moiety and the part(s) of the effector moiety involved in its physiological activity. Site-specific attachment of a variety of effector moieties may be performed using techniques known in the art, such as the use of bivalent cross-linking reagents and/or other chemical conjugation techniques.

### PEGylation by DNL

In a preferred method, the target to be PEGylated is linked to a DDD sequence to generate the DDD module. A PEG reagent of a desirable molecular size is derivatized with a related AD sequence and the resulting PEG-AD module is combined with the DDD module to produce the PEGylated conjugate that consists of a single PEG tethered site-specifically to two copies of the effector moiety via the disulfide bonds formed between DDD and AD. The PEG reagents may be capped at one end with a methoxy group (m-PEG), can be linear or branched, and may contain one of the following functional groups: propionic aldehyde, butyric aldehyde, ortho-pyridylthioester (OPTE), N-hydroxysuccinimide (NHS), thiazolidine-2-thione, succinimidyl carbonate (SC), maleimide, or ortho-pyridyldisulfide (OPPS). Among the effector moieties that may be of interest for PEGylation are enzymes, cytokines, chemokines, growth factors, peptides, aptamers, hemoglobins, antibodies and antibody fragments. The method is not limiting and a wide variety of agents may be PEGylated using the disclosed methods and compositions. PEG of various sizes and derivatized with a variety of reactive moieties may be obtained from commercial sources as discussed in more detail in the Examples below.

### Cytokines and Other Immunomodulators

It is disclosed that the effector moiety to be PEGylated is an immunomodulator. An immunomodulator is an agent that when present, alters, suppresses or stimulates the body's immune system. Immunomodulators of use may include a cytokine, a stem cell growth factor, a lymphotoxin, a hematopoietic factor, a colony stimulating factor (CSF), an interferon (IFN), erythropoietin, thrombopoietin and a combination thereof. Specifically useful are lymphotoxins such as tumor necrosis factor (TNF), hematopoietic factors, such as interleukin (IL), colony stimulating factor, such as granulocyte-colony stimulating factor (G-CSF) or granulocyte macrophage-colony stimulating factor (GM-CSF), interferon, such as interferons-α, -β or -γ, and stem cell growth factor, such as that designated "S1 factor".

It is more specifically disclosed that the effector moieties to be PEGylated are cytokines, such as lymphokines, monokines, growth factors and traditional polypeptide hormones. Included among the cytokines are growth hormones such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; prostaglandin, fibroblast growth factor; prolactin; placental lactogen, OB protein; tumor necrosis factor-α and - β; mullerian-inhibiting substance; mouse gonadotropin-associated peptide; inhibin; activin; vascular endothelial growth factor; integrin; thrombopoietin (TPO); nerve growth factors such as NGF-β; platelet-growth factor; transforming growth factors (TGFs) such as TGF- α and TGF- β; insulin-like growth factor-I and -II; erythropoietin (EPO); osteoinductive factors; interferons such as interferon-α, -β, and -γ; colony stimulating factors (CSFs) such as macrophage-CSF (M-CSF); interleukins (ILs) such as IL-1, IL-1α, IL-2, IL-3, IL-4, IL-5, IL-6, IL-7, IL-8, IL-9, IL-10, IL-11, IL-12; IL-13, IL-14, IL-15, IL-16, IL-17, IL-18, IL-21, IL-25, LIF, kit-ligand or FLT-3, angiostatin, thrombospondin, endostatin, tumor necrosis factor (TNF, such as TNF-α) and LT. The PEGylation of exemplary cytokines is described below in Examples 2 through 12.

The amino acid sequences of protein or peptide immunomodulators, such as cytokines, are well known in the art and any such known sequences may be used in the practice of the instant invention. The skilled artisan is aware of numerous sources of public information on cytokine sequence. For example, the NCBI database contains both protein and encoding nucleic acid sequences for a large number of cytokines and immunomodulators, such as erythropoietin (GenBank NM 000799), IL-1 beta (GenPept AAH08678), GM-CSF (GenPept AAA52578), TNF-α (GenPept CAA26669) and virtually any of the peptide or protein immunomodulators listed above. It is a matter of routine for the skilled artisan to identify an appropriate amino acid and/or nucleic acid sequence for essentially any protein or peptide effector moiety of interest.

### Antibodies and Antibody Fragments

It is also disclosed that antibodies or antigen-binding fragments of antibodies may be PEGylated. Antigen-binding antibody fragments are well known in the art, such as F(ab')₂, F(ab)₂, Fab', Fab, Fv, scFv and the like, and any such known fragment may be used. As used herein, an antigen-binding antibody fragment refers to any fragment of an antibody that binds with the same antigen that is recognized by the intact or parent antibody. Techniques for preparing AD and/or DDD conjugates of virtually any antibody or fragment of interest are known (e.g., U.S. Patent Application Serial No. 11/633,729).

An antibody or fragment thereof may be used which is not conjugated to a therapeutic agent - referred to as a "naked" antibody or fragment thereof. In alternative embodiments, antibodies or fragments may be conjugated to one or more therapeutic and/or diagnostic agents. A wide variety of such therapeutic and diagnostic agents are known in the art, as discussed in more detail below, and any such known therapeutic or diagnostic agent may be used.

Techniques for preparing monoclonal antibodies against virtually any target antigen are well known in the art. *See*, for example, Kohler and Milstein, Nature 256: 495 (1975), and Coligan et al. (eds.), CURRENT PROTOCOLS IN IMMUNOLOGY, VOL. 1, pages 2.5.1-2.6.7 (John Wiley & Sons 1991). Briefly, monoclonal antibodies can be obtained by injecting mice with a composition comprising an antigen, removing the spleen to obtain B-lymphocytes, fusing the B-lymphocytes with myeloma cells to produce hybridomas, cloning the hybridomas, selecting positive clones which produce antibodies to the antigen, culturing the clones that produce antibodies to the antigen, and isolating the antibodies from the hybridoma cultures.

MAbs can be isolated and purified from hybridoma cultures by a variety of well-established techniques. Such isolation techniques include affinity chromatography with Protein-A Sepharose, size-exclusion chromatography, and ion-exchange chromatography. See, for example, Coligan at pages 2.7.1-2.7.12 and pages 2.9.1-2.9.3. Also, see Baines et al., "Purification of Immunoglobulin G (IgG)," in METHODS IN MOLECULAR BIOLOGY, VOL. 10, pages 79-104 (The Humana Press, Inc. 1992).

After the initial raising of antibodies to the immunogen, the antibodies can be sequenced and subsequently prepared by recombinant techniques. Humanization and chimerization of murine antibodies and antibody fragments are well known to those skilled in the art. The use of antibody components derived from humanized, chimeric or human antibodies obviates potential problems associated with the immunogenicity of murine constant regions.

### Chimeric Antibodies

A chimeric antibody is a recombinant protein in which the variable regions of a human antibody have been replaced by the variable regions of, for example, a mouse antibody, including the complementarity-determining regions (CDRs) of the mouse antibody. Chimeric antibodies exhibit decreased immunogenicity and increased stability when administered to a subject. General techniques for cloning murine immunoglobulin variable domains are disclosed, for example, in Orlandi et al., Proc. Nat'l Acad. Sci. USA 86: 3833 (1989). Techniques for constructing chimeric antibodies are well known to those of skill in the art. As an example, Leung et al., Hybridoma 13:469 (1994), produced an LL2 chimera by combining DNA sequences encoding the V_{κ} and V_{H} domains of murine LL2, an anti-CD22 monoclonal antibody, with respective human κ and IgG₁ constant region domains.

### Humanized Antibodies

Techniques for producing humanized MAbs are well known in the art (see, e.g., Jones et al., Nature 321: 522 (1986), Riechmann et al., Nature 332: 323 (1988), Verhoeyen et al., Science 239: 1534 (1988), Carter et al., Proc. Nat'l Acad. Sci. USA 89: 4285 (1992), Sandhu, Crit. Rev. Biotech. 12: 437 (1992), and Singer et al., J Immun. 150: 2844 (1993)). A chimeric or murine monoclonal antibody may be humanized by transferring the mouse CDRs from the heavy and light variable chains of the mouse immunoglobulin into the corresponding variable domains of a human antibody. The mouse framework regions (FR) in the chimeric monoclonal antibody are also replaced with human FR sequences. As simply transferring mouse CDRs into human FRs often results in a reduction or even loss of antibody affinity, additional modification might be required in order to restore the original affinity of the murine antibody. This can be accomplished by the replacement of one or more some human residues in the FR regions with their murine counterparts to obtain an antibody that possesses good binding affinity to its epitope. See, for example, Tempest et al., Biotechnology 9:266 (1991) and Verhoeyen et al., Science 239: 1534 (1988). Generally, those human FR amino acid residues that differ from their murine counterparts and are located close to or touching one or more CDR amino acid residues would be candidates for substitution.

### Human Antibodies

Methods for producing fully human antibodies using either combinatorial approaches or transgenic animals transformed with human immunoglobulin loci are known in the art (*e*.*g*., Mancini et al., 2004, New Microbiol. 27:315-28; Conrad and Scheller, 2005, Comb. Chem. High Throughput Screen. 8:117-26; Brekke and Loset, 2003, Curr. Opin. Phamacol. 3:544-50). A fully human antibody also can be constructed by genetic or chromosomal transfection methods, as well as phage display technology, all of which are known in the art. See for example, McCafferty et al., Nature 348:552-553 (1990). Such fully human antibodies are expected to exhibit even fewer side effects than chimeric or humanized antibodies and to function *in vivo* as essentially endogenous human antibodies. In certain embodiments, the claimed methods and procedures may utilize human antibodies produced by such techniques.

In one alternative, the phage display technique may be used to generate human antibodies (*e.g*., Dantas-Barbosa et al., 2005, Genet. Mol. Res. 4:126-40). Human antibodies may be generated from normal humans or from humans that exhibit a particular disease state, such as cancer (Dantas-Barbosa et al., 2005). The advantage to constructing human antibodies from a diseased individual is that the circulating antibody repertoire may be biased towards antibodies against disease-associated antigens.

In one non-limiting example of this methodology, Dantas-Barbosa et al. (2005) constructed a phage display library of human Fab antibody fragments from osteosarcoma patients. Generally, total RNA was obtained from circulating blood lymphocytes (*Id*). Recombinant Fab were cloned from the µ, γ and κ chain antibody repertoires and inserted into a phage display library (*Id.*). RNAs were converted to cDNAs and used to make Fab cDNA libraries using specific primers against the heavy and light chain immunoglobulin sequences (Marks et al., 1991, J. Mol. Biol. 222:581-97). Library construction was performed according to Andris-Widhopf et al. (2000, In: Phage Display Laboratory Manual, Barbas et al. (eds), 1st edition, Cold Spring Harbor Laboratory Press, Cold Spring Harbor, NY pp. 9.1 to 9.22). The final Fab fragments were digested with restriction endonucleases and inserted into the bacteriophage genome to make the phage display library. Such libraries may be screened by standard phage display methods, as known in the art.

Phage display can be performed in a variety of formats, for their review, see e.g. Johnson and Chiswell, Current Opinion in Structural Biology 3:5564-571 (1993). Human antibodies may also be generated by *in vitro* activated B-cells. See U.S. Patent Nos. 5,567,610 and 5,229,275. The skilled artisan will realize that these techniques are exemplary and any known method for making and screening human antibodies or antibody fragments may be utilized.

In another alternative, transgenic animals that have been genetically engineered to produce human antibodies may be used to generate antibodies against essentially any immunogenic target, using standard immunization protocols. Methods for obtaining human antibodies from transgenic mice are disclosed by Green et al., Nature Genet. 7:13 (1994), Lonberg et al., Nature 368:856 (1994), and Taylor et al., Int. Immun. 6:579 (1994). A non-limiting example of such a system is the XenoMouse® (*e*.*g*., Green et al., 1999, J. Immunol. Methods 231:11-23) from Abgenix (Fremont, CA). In the XenoMouse® and similar animals, the mouse antibody genes have been inactivated and replaced by functional human antibody genes, while the remainder of the mouse immune system remains intact.

The XenoMouse® was transformed with germline-configured YACs (yeast artificial chromosomes) that contained portions of the human IgH and Igkappa loci, including the majority of the variable region sequences, along accessory genes and regulatory sequences. The human variable region repertoire may be used to generate antibody producing B-cells, which may be processed into hybridomas by known techniques. A XenoMouse® immunized with a target antigen will produce human antibodies by the normal immune response, which may be harvested and/or produced by standard techniques discussed above. A variety of strains of XenoMouse® are available, each of which is capable of producing a different class of antibody. Transgenically produced human antibodies have been shown to have therapeutic potential, while retaining the pharmacokinetic properties of normal human antibodies (Green et al., 1999). The skilled artisan will realize that the claimed compositions and methods are not limited to use of the XenoMouse® system but may utilize any transgenic animal that has been genetically engineered to produce human antibodies.

### Antibody Fragments

Antibody fragments which recognize specific epitopes can be generated by known techniques. Antibody fragments are antigen binding portions of an antibody, such as F(ab')₂, Fab', F(ab)₂, Fab, Fv, sFv and the like. F(ab')₂ fragments can be produced by pepsin digestion of the antibody molecule and Fab' fragments can be generated by reducing disulfide bridges of the F(ab')₂ fragments. Alternatively, Fab' expression libraries can be constructed (Huse et al., 1989, Science, 246:1274-1281) to allow rapid and easy identification of monoclonal Fab' fragments with the desired specificity. F(ab)₂ fragments may be generated by papain digestion of an antibody and Fab fragments obtained by disulfide reduction.

A single chain Fv molecule (scFv) comprises a VL domain and a VH domain. The VL and VH domains associate to form a target binding site. These two domains are further covalently linked by a peptide linker (L). Methods for making scFv molecules and designing suitable peptide linkers are described in US Patent No. 4,704,692, US Patent No. 4,946,778, R. Raag and M. Whitlow, "Single Chain Fvs." FASEB Vol 9:73-80 (1995) and R.E. Bird and B.W. Walker, "Single Chain Antibody Variable Regions," TIBTECH, Vol 9: 132-137 (1991).

An antibody fragment can be prepared by proteolytic hydrolysis of the full length antibody or by expression in *E. coli* or another host of the DNA coding for the fragment. An antibody fragment can be obtained by pepsin or papain digestion of full length antibodies by conventional methods. These methods are described, for example, by Goldenberg, U.S. Patent Nos. 4,036,945 and 4,331,647 and references contained therein. Also, see Nisonoff et al., Arch Biochem. Biophys. 89: 230 (1960); Porter, Biochem. J 73: 119 (1959), Edelman et al., in METHODS IN ENZYMOLOGY VOL. 1, page 422 (Academic Press 1967), and Coligan at pages 2.8.1-2.8.10 and 2.10.-2.10.4.

### Known Antibodies

Antibodies of use may be commercially obtained from a wide variety of known sources. For example, a variety of antibody secreting hybridoma lines are available from the American Type Culture Collection (ATCC, Manassas, VA). A large number of antibodies against various disease targets, including but not limited to tumor-associated antigens, have been deposited at the ATCC and/or have published variable region sequences and are available for use in the claimed methods and compositions. See, e.g., U.S. Patent Nos. 7,312,318; 7,282,567; 7,151,164; 7,074,403; 7,060,802; 7,056,509; 7,049,060; 7,045,132; 7,041,803; 7,041,802; 7,041,293; 7,038,018; 7,037,498; 7,012,133; 7,001,598; 6,998,468; 6,994,976; 6,994,852; 6,989,241; 6,974,863; 6,965,018; 6,964,854; 6,962,981; 6,962,813; 6,956,107; 6,951,924; 6,949,244; 6,946,129; 6,943,020; 6,939,547; 6,921,645; 6,921,645; 6,921,533; 6,919,433; 6,919,078; 6,916,475; 6,905,681; 6,899,879; 6,893,625; 6,887,468; 6,887,466; 6,884,594; 6,881,405; 6,878,812; 6,875,580; 6,872,568; 6,867,006; 6,864,062; 6,861,511; 6,861,227; 6,861,226; 6,838,282; 6,835,549; 6,835,370; 6,824,780; 6,824,778; 6,812,206; 6,793,924; 6,783,758; 6,770,450; 6,767,711; 6,764,688; 6,764,681; 6,764,679; 6,743,898; 6,733,981; 6,730,307; 6,720,15; 6,716,966; 6,709,653; 6,693,176; 6,692,908; 6,689,607; 6,689,362; 6,689,355; 6,682,737; 6,682,736; 6,682,734; 6,673,344; 6,653,104; 6,652,852; 6,635,482; 6,630,144; 6,610,833; 6,610,294; 6,605,441; 6,605,279; 6,596,852; 6,592,868; 6,576,745; 6,572;856; 6,566,076; 6,562,618; 6,545,130; 6,544,749; 6,534,058; 6,528,625; 6,528,269; 6,521,227; 6,518,404; 6,511,665; 6,491,915; 6,488,930; 6,482,598; 6,482,408; 6,479,247; 6,468,531; 6,468,529; 6,465,173; 6,461,823; 6,458,356; 6,455,044; 6,455,040, 6,451,310; 6,444,206' 6,441,143; 6,432,404; 6,432,402; 6,419,928; 6,413,726; 6,406,694; 6,403,770; 6,403,091; 6,395,276; 6,395,274; 6,387,350; 6,383,759; 6,383,484; 6,376,654; 6,372,215; 6,359,126; 6,355,481; 6,355,444; 6,355,245; 6,355,244; 6,346,246; 6,344,198; 6,340,571; 6,340,459; 6,331,175; 6,306,393; 6,254,868; 6,187,287; 6,183,744; 6,129,914; 6,120,767; 6,096,289; 6,077,499; 5,922,302; 5,874,540; 5,814,440; 5,798,229; 5,789,554; 5,776,456; 5,736,119; 5,716,595; 5,677,136; 5,587,459; 5,443,953, 5,525,338. These are exemplary only and a wide variety of other antibodies and their hybridomas are known in the art. The skilled artisan will realize that antibody sequences or antibody-secreting hybridomas against almost any disease-associated antigen may be obtained by a simple search of the ATCC, NCBI and/or USPTO databases for antibodies against a selected disease-associated target of interest. The antigen binding domains of the cloned antibodies may be amplified, excised, ligated into an expression vector, transfected into an adapted host cell and used for protein production, using standard techniques well known in the art.

### Therapeutic Agents

In alternative embodiments, therapeutic agents such as cytotoxic agents, anti-angiogenic agents, pro-apoptotic agents, antibiotics, hormones, hormone antagonists, chemokines, drugs, prodrugs, toxins, enzymes or other agents may be PEGylated as described herein. PEGylated forms of therapeutic agents have been disclosed, for example, for SN38 (Zhao et al., Bioconjug Chem 2008, 10:849-59), uricase (Biggers & Scheinfeld, Curr Opin Investig Drugs 2008, 9:422-29), docetaxel (Liu et al., 2008, J Pharm Sci 97:3274-90) and camptothecin (Haverstick et al., 2007, Bioconjug Chem 18:2115-21). Drugs of use may possess a pharmaceutical property selected from the group consisting of antimitotic, antikinase, alkylating, antimetabolite, antibiotic, alkaloid, anti-angiogenic, pro-apoptotic agents and combinations thereof.

Exemplary drugs of use may include 5-fluorouracil, aplidin, azaribine, anastrozole, anthracyclines, bendamustine, bleomycin, bortezomib, bryostatin-1, busulfan, calicheamycin, camptothecin, carboplatin, 10-hydroxycamptothecin, carmustine, celebrex, chlorambucil, cisplatin (CDDP), Cox-2 inhibitors, irinotecan (CPT-11), SN-38, carboplatin, cladribine, camptothecans, cyclophosphamide, cytarabine, dacarbazine, docetaxel, dactinomycin, daunorubicin, doxorubicin, 2-pyrrolinodoxorubicine (2P-DOX), cyano-morpholino doxorubicin, doxorubicin glucuronide, epirubicin glucuronide, estramustine, epidophyllotoxin, estrogen receptor binding agents, etoposide (VP 16), etoposide glucuronide, etoposide phosphate, floxuridine (FUdR), 3',5'-O-dioleoyl-FudR (FUdR-dO), fludarabine, flutamide, farnesyl-protein transferase inhibitors, gemcitabine, hydroxyurea, idarubicin, ifosfamide, L-asparaginase, lenolidamide, leucovorin, lomustine, mechlorethamine, melphalan, mercaptopurine, 6-mercaptopurine, methotrexate, mitoxantrone, mithramycin, mitomycin, mitotane, navelbine, nitrosurea, plicomycin, procarbazine, paclitaxel, pentostatin, PSI-341, raloxifene, semustine, streptozocin, tamoxifen, taxol, temazolomide (an aqueous form of DTIC), transplatinum, thalidomide, thioguanine, thiotepa, teniposide, topotecan, uracil mustard, vinorelbine, vinblastine, vincristine and vinca alkaloids.

Toxins of use may include ricin, abrin, alpha toxin, saporin, ribonuclease (RNase), e.g., onconase, DNase I, *Staphylococcal* enterotoxin-A, pokeweed antiviral protein, gelonin, diphtheria toxin, *Pseudomonas* exotoxin, and *Pseudomonas* endotoxin.

Chemokines of use may include RANTES, MCAF, MIP1-alpha, MIP1-Beta and IP-10.

In certain embodiments, anti-angiogenic agents, such as angiostatin, baculostatin, canstatin, maspin, anti-VEGF antibodies, anti-PIGF peptides and antibodies, anti-vascular growth factor antibodies, anti-Flk-1 antibodies, anti-Flt-1 antibodies and peptides, anti-Kras antibodies, anti-cMET antibodies, anti-MIF (macrophage migration-inhibitory factor) antibodies, laminin peptides, fibronectin peptides, plasminogen activator inhibitors, tissue metalloproteinase inhibitors, interferons, interleukin-12, IP-10, Gro-β, thrombospondin, 2-methoxyoestradiol, proliferin-related protein, carboxiamidotriazole, CM101, Marimastat, pentosan polysulphate, angiopoietin-2, interferon-alpha, herbimycin A, PNU145156E, 16K prolactin fragment, Linomide, thalidomide, pentoxifylline, genistein, TNP-470, endostatin, paclitaxel, accutin, angiostatin, cidofovir, vincristine, bleomycin, AGM-1470, platelet factor 4 or minocycline may be of use.

Other useful therapeutic agents may comprise oligonucleotides, especially antisense oligonucleotides that preferably are directed against oncogenes and oncogene products, such as bcl-2 or p53.

### Conjugation

A variety of techniques and compositions for conjugation of small molecules to proteins or peptides, such as AD or DDD peptides, are known and may be used. Therapeutic agents can be attached, for example to reduced SH groups and/or to carbohydrate side chains. A therapeutic agent can be attached to a reduced protein or peptide comprising a cysteine residue via disulfide bond formation. Alternatively, such agents can be attached using a heterobifunctional cross-linker, such as *N*-succinyl 3-(2-pyridyldithio)propionate (SPDP). Yu et al., Int. J. Cancer 56: 244 (1994). General techniques for such conjugation are well-known in the art. See, for example, Wong, CHEMISTRY OF PROTEIN CONJUGATION AND CROSS-LINKING (CRC Press 1991); Upeslacis et al., "Modification of Antibodies by Chemical Methods," in MONOCLONAL ANTIBODIES: PRINCIPLES AND APPLICATIONS, Birch et al. (eds.), pages 187-230 (Wiley-Liss, Inc. 1995); Price, "Production and Characterization of Synthetic Peptide-Derived Antibodies," in MONOCLONAL ANTIBODIES: PRODUCTION, ENGINEERING AND CLINICAL APPLICATION, Ritter et al. (eds.), pages 60-84 (Cambridge University Press 1995).

### Therapeutic Use

The compositions described herein are particularly useful for treatment of various disease states. In preferred embodiments, the diseases may be autoimmune diseases or cancer, such as hematopoietic cancers or solid tumors. Exemplary non-limiting diseases that may be treated using the disclosed compositions and methods include indolent forms of B-cell lymphomas, aggressive forms of B-cell lymphomas, non-Hodgkin's lymphoma, multiple myeloma, chronic lymphatic leukemias, acute lymphatic leukemias, acute myelogenous leukemia, chronic lymphocytic leukemia, chronic myelogenous leukemia, Hodgkin's lymphoma, Waldenström's macroglobulinemia, as well as GVHD, cryoglobulinemia, hemolytic anemia, allosensitization, and organ transplant rejection. Also included are class III autoimmune diseases such as immune-mediated thrombocytopenias, such as acute idiopathic thrombocytopenic purpura and chronic idiopathic thrombocytopenic purpura, dermatomyositis, Sjögren's syndrome, multiple sclerosis, Sydenham's chorea, myasthenia gravis, systemic lupus erythematosus, lupus nephritis, rheumatic fever, rheumatoid arthritis, polyglandular syndromes, bullous pemphigoid, diabetes mellitus, Henoch-Schönlein purpura, post-streptococcal nephritis, erythema nodosum, Takayasu's arteritis, Addison's disease, sarcoidosis, ulcerative colitis, erythema multiforme, IgA nephropathy, polyarteritis nodosa, ankylosing spondylitis, Goodpasture's syndrome, thromboangitis obliterans, primary biliary cirrhosis, Hashimoto's thyroiditis, thyrotoxicosis, scleroderma, chronic active hepatitis, polymyositis/dermatomyositis, polychondritis, pemphigus vulgaris, Wegener's granulomatosis, membranous nephropathy, amyotrophic lateral sclerosis, tabes dorsalis, giant cell arteritis/polymyalgia, pernicious anemia, rapidly progressive glomerulonephritis and fibrosing alveolitis.

Solid tumors that may be treated include neuroblastoma, malignant melanoma, breast, ovarian, cervical, uterine, endometrial, prostatic, lung, kidney, colorectal, gastric, bladder, glioma, sarcoma, brain, esophageal, epithelial, osteosarcoma, testicular, liver and pancreatic cancers.

### Kits

Also disclosed are kits containing components suitable for treating or diagnosing diseased tissue in a patient. Exemplary kits may contain at least PEGylated therapeutic agent as described herein. If the composition containing components for administration is not formulated for delivery via the alimentary canal, such as by oral delivery, a device capable of delivering the kit components through some other route may be included. One type of device, for applications such as parenteral delivery, is a syringe that is used to inject the composition into the body of a subject. Inhalation devices may also be used. In certain embodiments, a PEGylated therapeutic agent may be provided in the form of a prefilled syringe or autoinjection pen containing a sterile, liquid formulation or lyophilized preparation.

The kit components may be packaged together or separated into two or more containers. In some embodiments, the containers may be vials that contain sterile, lyophilized formulations of a composition that are suitable for reconstitution. A kit may also contain one or more buffers suitable for reconstitution and/or dilution of other reagents. Other containers that may be used include, but are not limited to, a pouch, tray, box, tube, or the like. Kit components may be packaged and maintained sterilely within the containers. Another component that can be included is instructions to a person using a kit for its use.

### EXAMPLES

The following examples are provided to illustrate, but not to limit, the claims of the present invention.

### Example 1. Generation of PEG-AD2 modules

### Synthesis of IMP350

CGQIEYLAKQIVDNAIQQAGC(SS-tbu)-NH₂ (SEQ ID NO:1) MH⁺2354

IMP350, incorporating the sequence of AD2, was made on a 0.1 mmol scale with Sieber Amide resin using Fmoc methodology on a Protein Technologies PS3 peptide synthesizer. Starting from the C-terminus the protected amino acids used were Fmoc-Cys(t-Buthio)-OH, Fmoc-Gly-OH, Fmoc-Ala-OH, Fmoc-Gln(Trt)-OH, Fmoc-Gln(Trt)-OH, Fmoc-Ile-OH, Fmoc-Ala-OH, Fmoc-Asn(Trt)-OH, Fmoc-Asp(OBut)-OH, Fmoc-Val-OH, Fmoc-, Ile-OH, Fmoc-Gln(Trt)-OH, Fmoc-Lys(Boc)-OH, Fmoc-Ala-OH, Fmoc-Leu-OH, Fmoc-Tyr(But)-OH, Fmoc-Glu(OBut)-OH, Fmoc-Ile-OH, Fmoc-Gln(Trt)-OH, Fmoc-Gly-OH and Fmoc-Cys(Trt)-OH. The peptide was cleaved from the resin and purified by reverse phase (RP)-HPLC.

### Synthesis of PEG₂₀-IMP350

IMP350 (0.0104 g) was mixed with 0.1022 g of mPEG-OPTE (20kDa, Nektar Therapeutics) in 7 mL of 1 M Tris buffer at pH 7.81. Acetonitrile, 1 mL, was then added to dissolve some suspended material. The reaction was stirred at room temperature for 3 h and then 0.0527 g of TCEP was added along with 0.0549 g of cysteine. The reaction mixture was stirred for 1.5 h and then purified on a PD-10 desalting column, which was equilibrated with 20% methanol in water. The sample was eluted, frozen and lyophilized to obtain 0.0924 g of crude PEG₂₀-IMP350 (MH+ 23508 by MALDI).

### Synthesis of IMP360

CGQIEYLAKQIVDNAIQQAGC(SS-tbu)G-EDANS (SEQ ID NO:1) MH⁺ 2660

IMP 360, incorporating the AD2 sequence, was synthesized on a 0.1 mmol scale with Fmoc-Gly-EDANS resin using Fmoc methodology on a Protein Technologies PS3 peptide synthesizer. The Fmoc-Gly-OH was added to the resin manually using 0.23 g of Fmoc-Gly-OH, 0.29 g of HATU, 26 µL of DIEA, 7.5 mL of DMF and 0.57 g of EDANS resin (Nova Biochem). The reagents were mixed and added to the resin. The reaction was mixed at room temperature for 2.5 hr and the resin was washed with DMF and IPA to remove the excess reagents. Starting from the C-terminus the protected amino acids used were Fmoc-Cys(t-Buthio)-OH, Fmoc-Gly-OH, Fmoc-Ala-OH, Fmoc-Gln(Trt)-OH, Fmoc-Gln(Trt)-OH, Fmoc-Ile-OH, Fmoc-Ala-OH, Fmoc-Asn(Trt)-OH, Fmoc-Asp(OBut)-OH, Fmoc-Val-OH, Fmoc-Ile-OH, Fmoc-Gln(Trt)-OH, Fmoc-Lys(Boc)-OH, Fmoc-Ala-OH, Fmoc-Leu-OH, Fmoc-Tyr(But)-OH, Fmoc-Glu(OBut)-OH, Fmoc-Ile-OH, Fmoc-Gln(Trt)-OH, Fmoc-Gly-OH and Fmoc-Cys(Trt)-OH. The peptide was cleaved from the resin and purified by RP-HPLC.

### Synthesis of IMP362 (PEG₂₀-IMP360)

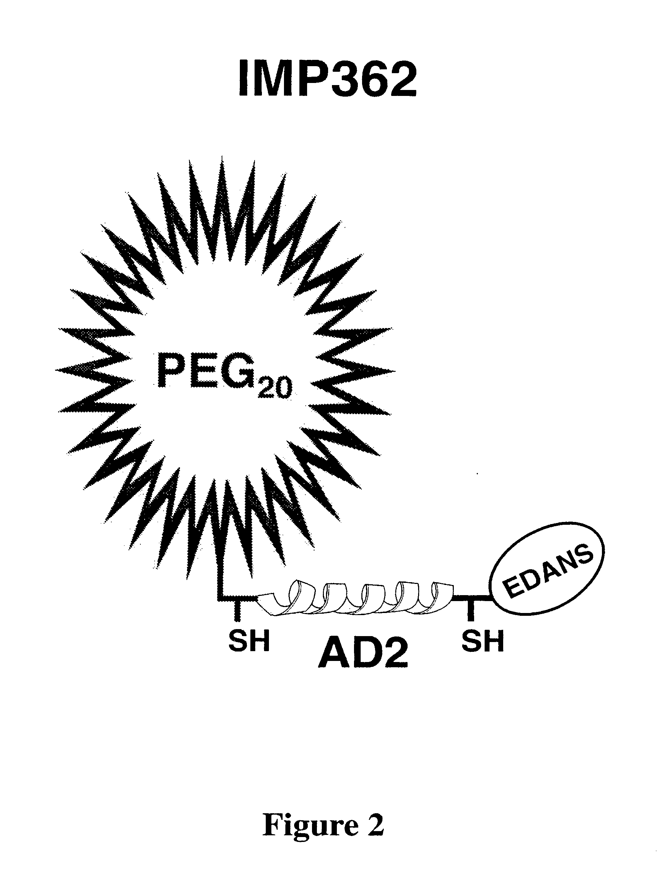

A cartoon diagram of IMP362 is provided in FIG. 2. For synthesis of IMP362, IMP360 (0.0115 g) was mixed with 0.1272 g of mPEG-OPTE (20kDa, Nektar Therapeutics) in 7 mL of 1 M tris buffer, pH 7.81. Acetonitrile (1 mL) was then added to dissolve some suspended material. The reaction was stirred at room temperature for 4 h and then 0.0410 g of TCEP was added along with 0.0431 g of cysteine. The reaction mixture was stirred for 1 h and purified on a PD-10 desalting column, which was equilibrated with 20 % methanol in water. The sample was eluted, frozen and lyophilized to obtain 0.1471 g of crude IMP362 (MH+ 23713).

### Synthesis of IMP413 (PEG₃₀-IMP360)

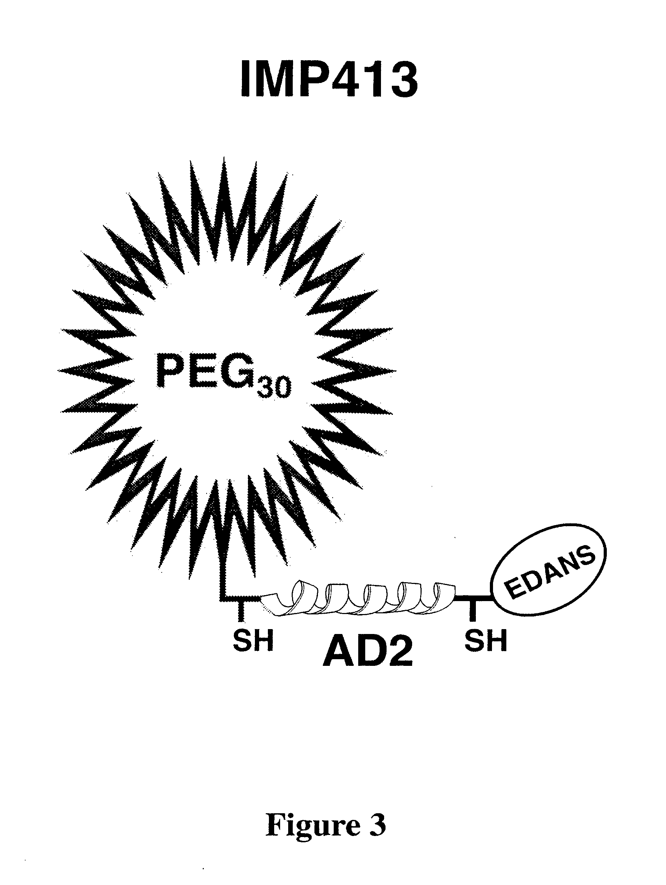

A cartoon diagram of IMP 413 is provided in FIG. 3. For synthesis of IMP 413, IMP 360 (0.0103 g) was mixed with 0.1601 g of mPEG-OPTE (30kDa, Nektar Therapeutics) in 7 mL of 1 M tris buffer at pH 7.81. Acetonitrile (1 mL) was then added to dissolve some suspended material. The reaction was stirred at room temperature for 4.5 h and then 0.0423 g of TCEP was added along with 0.0473 g of cysteine. The reaction mixture was stirred for 2 h followed by dialysis for two days. The dialyzed material was frozen and lyophilized to obtain 0.1552 g of crude IMP413 (MH⁺ 34499).

### Synthesis of IMP421

IMP 421 Ac-C-PEG₃-C(S-tBu)GQIEYLAKQIVDNAIQQAGC(S-tBu)G-NH₂ (SEQ ID NO:9)

The peptide IMP421, MH⁺ 2891 was made on NovaSyn® TGR resin (487.6 mg, 0.112 mmol) by adding the following amino acids to the resin in the order shown: Fmoc-Gly-OH, Fmoc-Cys(t-Buthio)-OH, Fmoc-Gly-OH, Fmoc-Ala-OH, Fmoc-Gln(Trt)-OH, Fmoc-Gln(Trt)-OH, Fmoc-Ile-OH, Fmoc-Ala-OH, Fmoc-Asn(Trt)-OH, Fmoc-Asp(OBut)-OH, Fmoc-Val-OH, Fmoc-Ile-OH, Fmoc-Gln(Trt)-OH, Fmoc-Lys(Boc)-OH, Fmoc-Ala-OH, Fmoc-Leu-OH, Fmoc-Tyr(But)-OH, Fmoc-Glu(OBut)-OH, Fmoc-Ile-OH, Fmoc-Gln(Trt)-OH, Fmoc-Gly-OH, Fmoc-Cys(t-Buthio)-OH, Fmoc-NH-PEG₃-COOH, Fmoc-Cys(Trt)-OH. The N-terminal amino acid was protected as an acetyl derivative. The peptide was then cleaved from the resin and purified by RP-HPLC to yield 32.7 mg of a white solid.

### Synthesis of IMP457

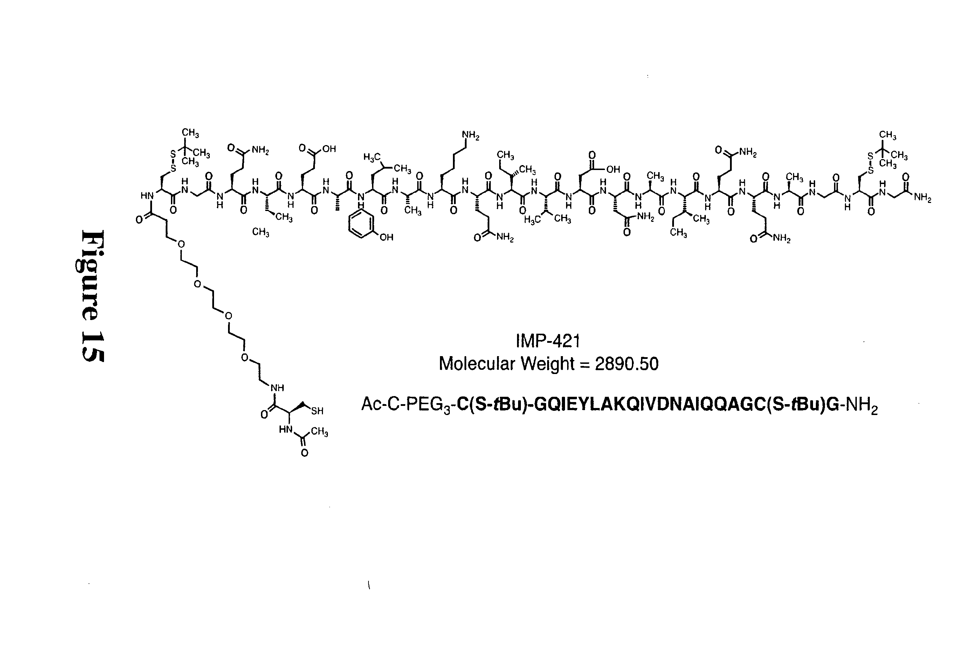

IMP 421 (SEQ ID NO:9, FIG. 15), incorporating the sequence of AD2, was synthesized by standard chemical means. To a solution of 15.2 mg (5.26 µmol) IMP 421 (F.W. 2890.50) and 274.5 mg (6.86 µmol) mPEG2-MAL-40K in 1 mL of acetonitrile was added 7 mL 1 M Tris pH 7.8 and allowed to react at room temperature for 3 h. The excess mPEG2-MAL-40K (FIG. 16) was quenched with 49.4 mg L-cysteine, followed by S-S-tBu deprotection over one hour with 59.1 mg TCEP. The reaction mixture was dialyzed overnight at 2-8 °C using two 3-12 mL capacity 10K Slide-A-Lyzer dialysis cassettes (4 ml into each cassette) into 5 L of 5 mM ammonium acetate, pH 5.0. Three more 5 L buffer changes of 5 mM ammonium acetate, pH 5.0 were made the next day with each dialysis lasting at least 2½ h. The purified product (19.4 mL) was transferred into two 20 mL scintillation vials, frozen and lyophilized to yield 246.7 mg of a white solid. MALDI-TOF gave results of mPEG2-MAL-40K 42,982 and IMP-457 45,500.

### Example 2. Generation of DDD module based on Interferon (IFN)-α2b

### Construction of IFN α2b-DDD2-pdHL2 for expression in mammalian cells

The cDNA sequence for IFN-α2b was amplified by PCR, resulting in a sequence comprising the following features, in which XbaI and BamHI are restriction sites, the signal peptide is native to IFN-α2b, and 6 His is a hexahistidine tag: Xbal---Signal peptide-IFNα2b ---6 His---BamHI. The resulting secreted protein consists of IFN-α2b fused at its C-terminus to a polypeptide consisting of SEQ ID NO:2.

PCR amplification was accomplished using a full length human IFNα2b cDNA clone (Invitrogen Ultimate ORF human clone cat# HORF01Clone ID IOH35221) as a template and the following oligonucleotides as primers:
IFNA2 Xba I Left
5'-TCTAGACACAGGACCTCATCATGGCCTTGACCTTTGCTTTACTGG-3' (SEQ ID NO:3)
IFNA2 BamHI right

The PCR amplimer was cloned into the pGemT vector (Promega). A DDD2-pdHL2 mammalian expression vector was prepared for ligation with IFN-α2b by digestion with XbaI and Bam HI restriction endonucleases. The IFN-α2b amplimer was excised from pGemT with Xbal and Bam HI and ligated into the DDD2-pdHL2 vector to generate the expression vector IFN-α2b-DDD2-pdHL2.

### Mammalian expression of IFN-α2b-DDD2

IFN-α2b-DDD2-pdHL2 was linearized by digestion with SalI enzyme and stably transfected into Sp/EEE myeloma cells by electroporation (see. e.g., U.S. Patent Application Serial No. 11/487,215, filed 7/14/06). Two clones were found to have detectable levels of IFN-α2b by ELISA. One of the two clones, designated 95, was adapted to growth in serum-free media without substantial decrease in productivity. The clone was subsequently amplified with increasing methotrexate (MTX) concentrations from 0.1 to 0.8 µM over five weeks. At this stage, it was sub-cloned by limiting dilution and the highest producing sub-clone (95-5) was expanded. The productivity of 95-5 grown in shake-flasks was estimated to be 2.5 mg/L using commercial rIFN-α2b (Chemicon IF007, Lot 06008039084) as a standard.

### Purification of IFN-α2b-DDD2 from batch cultures grown in roller bottles

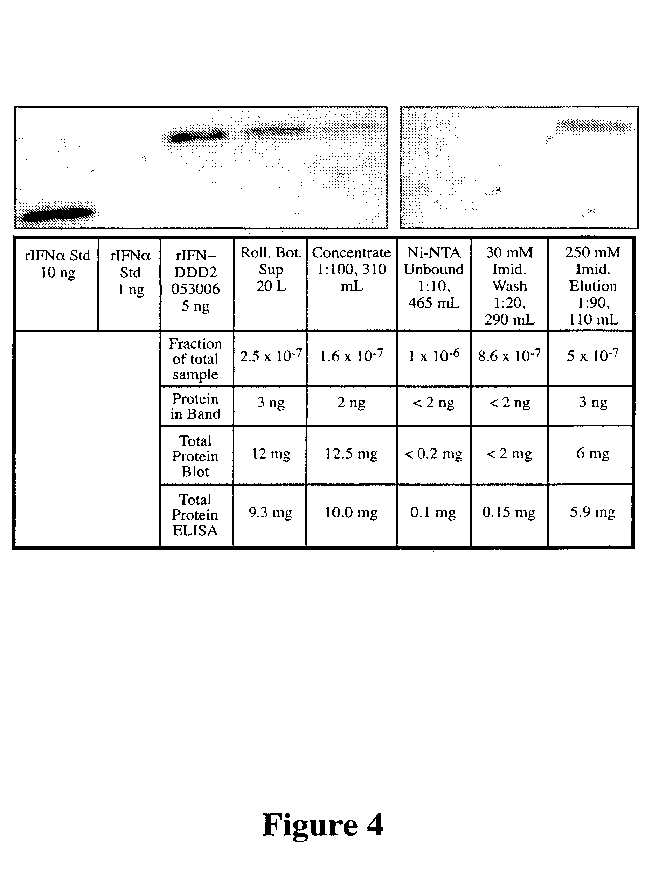

Clone 95-5 was expanded to 34 roller bottles containing a total of 20 L of serum-free Hybridoma SFM with 0.8 µM MTX and allowed to reach terminal culture. The supernatant fluid was clarified by centrifugation, filtered (0.2 µM). The filtrate was diafiltered into 1X Binding buffer (10 mM imidazole, 0.5 M NaCl, 50 mM NaH₂PO₄, pH 7.5) and concentrated to 310 mL in preparation for purification by immobilized metal affinity chromatography (IMAC). The concentrate was loaded onto a 30-mL Ni-NTA column, which was washed with 500 mL of 0.02% Tween 20 in 1X binding buffer and then 290 mL of 30 mM imidazole, 0.02% Tween 20, 0.5 M NaCl, 50 mM NaH₂PO₄, pH 7.5. The product was eluted with 110 mL of 250 mM imidazole, 0.02% Tween 20, 150 mM NaCl, 50 mM NaH₂PO₄, pH 7.5. Approximately 6 mg of IFNα2b-DDD2 was purified. FIG. 4 shows the results of an anti-IFNα immunoblot and ELISA used to quantify IFNα2b-DDD2.

### Characterization of IFN-α2b-DDD2

The purity of IFN-α2b-DDD2 was assessed by SDS-PAGE under reducing conditions (not shown). The Coomassie blue-stained gel showed that the batch produced from roller bottles was purer than an earlier batch (not shown). IFN-α2b-DDD2 was the most heavily stained band and accounted for approximately 50% of the total protein (not shown). The product resolved as a doublet with an Mᵣ of ∼26 kDa, which is consistent with the calculated MW of IFN-α2b-DDD2-SP (26 kDa). There was one major contaminant with a Mᵣ of 34 kDa and many faint contaminating bands (not shown).

### Example 3. Generation of PEGylated IFN-α2b by DNL

### Preparation and purification of α2b-362 (IFN-α2b-DDD2-IMP362)

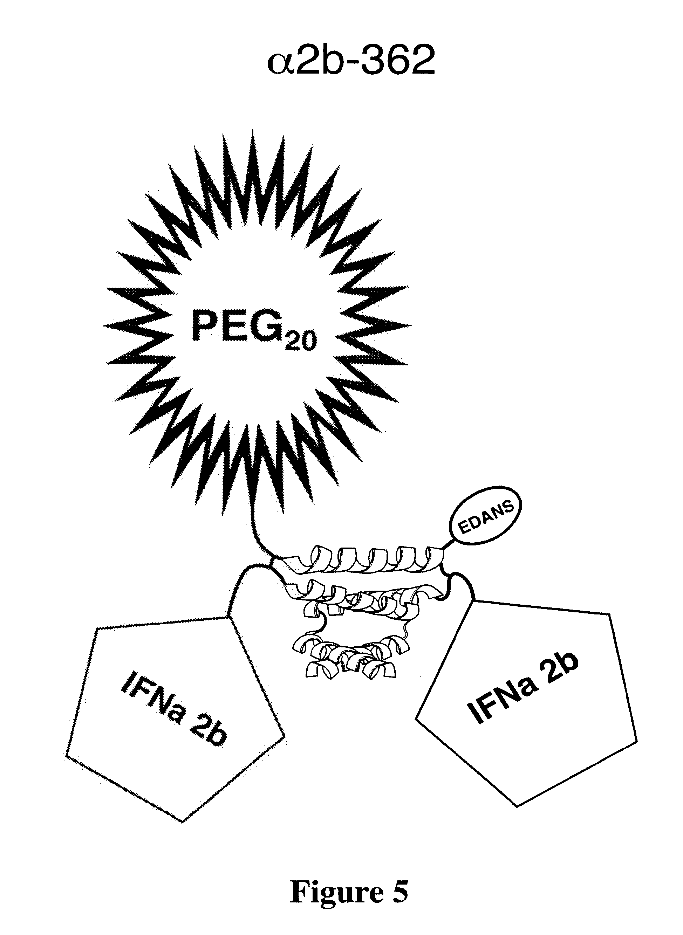

A cartoon drawing depicting the structure of α2b-362 having two copies of IFNα2b coupled to a 20 kDa PEG is provided in FIG. 5. A DNL reaction was performed by the addition of 11 mg of reduced and lyophilized IMP362 in 10-fold molar excess to 2.25 mg (3.5 ml) of IFN-α2b-DDD2 in 250 mM imidazole, 0.02% Tween 20, 150 mM NaCl, 1 mM EDTA, 50 mM NaH₂PO₄, pH 7.5. After 6 h at room temperature in the dark, the reaction mixture was dialyzed against CM Loading Buffer (150 mM NaCl, 20 mM NaAc, pH 4.5) at 4°C in the dark. The solution was loaded onto a 1-mL Hi-Trap CM-FF column (Amersham), which was pre-equilibrated with CM Loading buffer. After sample loading, the column was washed with CM loading buffer to baseline, followed by washing with 15 mL of 0.25 M NaCl, 20 mM NaAc, pH 4.5. The PEGylated product was eluted with 12.5 mL of 0.5 M NaCl, 20 mM NaAc, pH 4.5.

The conjugation process was analyzed by SDS-PAGE with Coomassie blue staining, fluorescence imaging and anti-IFNα immunoblotting (not shown). To normalize the samples for direct protein mass comparison, each fraction eluted from the CM-FF column was concentrated to 3.5 mL to match the reaction volume. Under non-reducing conditions, the Coomassie blue-stained gel revealed the presence of a major band at a Mᵣ of 110 kDa in the reaction mixture, which was absent in the unbound or 0.25 M NaCl wash fraction, but evident in the 0.5 M NaCl fraction (not shown). Fluorescence imaging, which was used to detect the EDANS tag on IMP362, demonstrated that the 110 kDa band contained IMP362 and the presence of excess IMP362 in the reaction mixture and the unbound fraction, which did not stain with Coomassie blue (not shown). Anti-IFNα immunoblotting confirmed the association of IFN-α2b with the 110 kDa band (not shown). These data together indicate that the DNL reaction resulted in the site-specific and covalent conjugation of IMP362 with a dimer of IFN-α2b. Under reducing conditions, which breaks the disulfide linkage, the components of the DNL structures were resolved (not shown). The calculated MW of α2b-362 was ∼75 kDa, which matches well the mass of 76,728 Da determined by MALDI TOF. The observed discrepancy between the calculated mass and the estimated Mr by SDS-PAGE is due to PEG, which is known to inflate the molecular size when PEGylated products are analyzed by SDS-PAGE or SE-HPLC. Overall, the DNL reaction resulted in a near quantitative yield of a homogeneous product that was > 90% pure after purification by cation-exchange chromatography (not shown).

### Preparation and purification of α2b-457 (IFN-α2b-DDD2-IMP457)

A DNL reaction was performed by the addition of 2.5 mg of reduced and lyophilized IMP457 in 10-fold molar excess to 1 mg (1.7 ml) of IFN-α2b-DDD2 in 250 mM imidazole, 0.02% Tween 20, 150 mM NaCl, 1 mM EDTA, 50 mM NaH₂PO₄, pH 7.5. After 60 h at room temperature, 1mM oxidized glutathione was added to the reaction mixture, which was then held for an additional 2 h. The mixture was diluted 1:20 with CM Loading Buffer (150 mM NaCl, 20 mM NaAc, pH 4.5) and titrated to pH 4.5 with acetic acid. The solution was loaded onto a 1-mL Hi-Trap CM-FF column (Amersham), which was pre-equilibrated with CM Loading Buffer. After sample loading, the column was washed with CM Loading Buffer to baseline, followed by washing with 15 mL of 0.25 M NaCl, 20 mM NaAc, pH 4.5. The PEGylated product was eluted with 20 mL of 0.5 M NaCl, 20 mM NaAc, pH 4.5. The a2b-457 was concentrated to 2 mL and diafiltered into 0.4 M PBS, pH 7.4. The final yield was approximately 1 mg of a2b-457 of >90% purity as determined by SDS-PAGE and IFNα ELISA.

### Preparation and purification of α2b-413 (IFN-α2b-DDD2-IMP413)

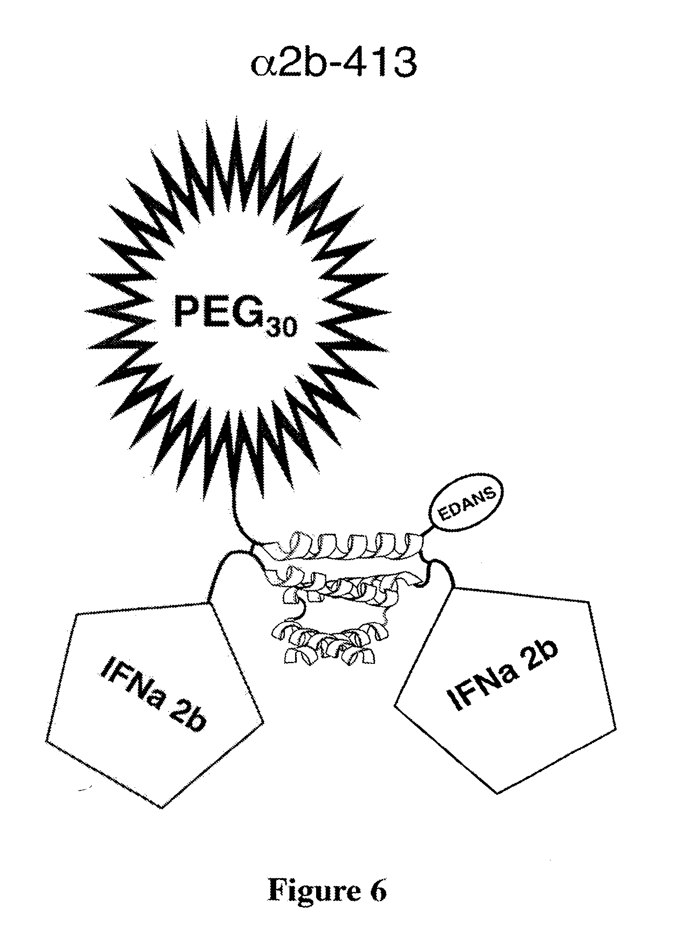

A cartoon drawing depicting the structure of α2b-413 having two copies of IFNα2b coupled to a 30 kDa PEG is provided in FIG. 6. α2b-413 was prepared as described immediately above using IMP413 instead of IMP362.

### Example 4. Evaluation of the in vitro potency of IFN-α2b-DDD2, α2b-362, and α2b-413

### In vitro anti-proliferative assay

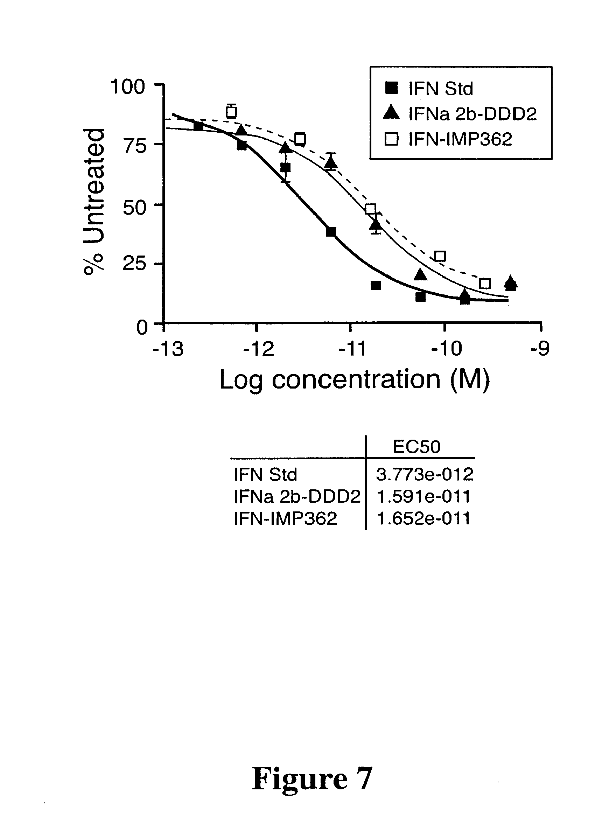

IFN-α2b-DDD2 and α2b-362 were assayed for inhibition of growth of Burkitt's lymphoma (Daudi) cells. Briefly, IFN-α2b standard (Chemicon IF007, Lot 06008039084), IFN-α2b-DDD2 (batch 010207) and α2b-362 (batch 010807) were each diluted to 500 pM in RPMI 1640 media supplemented with 10% FBS, from which three-fold serial dilutions in triplicate were made in 96-well tissue culture plates (50 µL sample/well). Daudi cells were diluted to 4 x 10⁵ cells/mL and 50 µL were added to each well (20K/well). The concentration range for each test reagent was 500 pM to 0.008 pM. After 4 days at 37°C, MTS dye was added to the plates (20 µL per well) and after 3 h the plates were read with an Envision plate reader (Perkin Elmer, Boston MA) at 490 nm. Dose-response curves were generated (FIG. 7) and 50% effective concentration (EC₅₀) values were obtained by sigmoidal fit non-linear regression using Graph Pad Prism software (Advanced Graphics Software, Encinitas, CA). The calculated EC₅₀ for IFNα2b-DDD2 and α2b-362 were similar (~ 16 pM) and about 5-fold less potent than the IFN-α2b standard (EC₅₀ ∼4 pM). In a similar experiment the α2b-413 had similar potency as α2b-362.

### Anti-viral assay

Duplicate samples were analyzed in a viral challenge assay using encephalomyocarditis (EMC) virus on A549 cells by an independent analytical laboratory (PBL Interferon Source, Piscataway, NJ). Plates were stained with crystal violet and the OD was measured by spectrophotometry on a 96-well plate reader following solubilization of the dye. The data were analyzed with Graph Pad Prizm software using a sigmoidal fit (variable slope) non-linear regression. The anti-viral titer was determined by comparison of EC₅₀ values with that of an IFNα standard. The specific anti-viral activities were calculated at 1.2 x 10⁸ U/mg and 8.8 x 10⁶ U/mg for α2b-362 and α2b-413, respectively.

### Example 5. In vivo evaluation of α2b-413 and a2b-362

### Pharmacokinetics

The study was performed in adult female Swiss-Webster mice (∼35 g). There were 4 different treatment groups of 2 mice each. Each reagent (test and control) was administered at equimolar protein doses (3 µg of rhuIFN-α2a, 5 µg of PEGINTRON™, 11 µg of α2b-362, and 13 µg of α2b-413) as a single bolus i.v. injection. Mice were bled *via* the retro-orbital method at various time-points (pre-dose, 5-min, 2-, 8-, 24-, 48-, 72-, 96-, and 168-h post-injection). The blood was allowed to clot, centrifuged, and the serum was isolated and stored at -70°C until assayed for IFN-α concentration and subsequent PK-analysis.

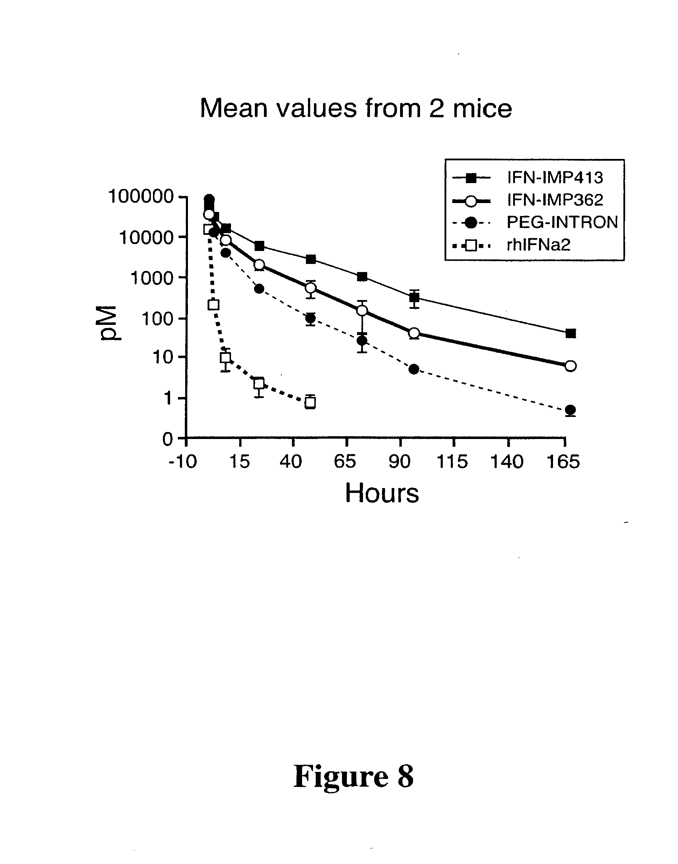

Concentrations of IFN-α in the serum samples were determined using a human interferon alpha ELISA kit following the manufacturer's instructions (PBL Interferon Source). Briefly, the serum samples were diluted appropriately according to the human IFN-α standard provided in the kit. An antibody coupled to the microtiter plate wells captured interferon. A second antibody was then used to reveal the bound interferon, which was quantified by anti-secondary antibody conjugated to horseradish peroxidase (HRP) following the addition of Tetramethyl benzidine (TMB) substrate. The plates were read at 450nm, and the results are shown in FIG. 8.

The PK properties of each agent are summarized in Table 1. As expected, rhIFN-α2a had the most rapid clearance from the blood of injected mice. Its clearance was approximately 3-fold faster than the PEGINTRON™ and more than 13-fold faster than the DNL-IFN reagents. The PEGINTRON™ was in turn cleared greater than 4-fold faster than α2b-362 or α2b-413. There was little difference in the elimination rates between α2b-362 and α2b-413.

In terms of mean residence time (MRT), there is a clear correlation with size among the various reagents. The 19-kDa rhIFN-α2a had a MRT that was 7-fold less than the 31 kDa PEGINTRON™ (0.7 h *versus* 5.1 h, respectively), which had a 2-fold lower MRT when compared to the 70 kDa α2b-362 (10.3 h). The MRT for the 80 kDa α2b-413 (21.7 h) was 2-fold longer than α2b-362. Finally, a test for bioequivalence showed that none of the reagents tested were the same in terms of PK, indicating that the differences are genuine (i.e., circulating half-life for α2b-413 > α2b-362 > PEGINTRON™ > rhIFN-α2a).

| Table 1. Blood Pharmacokinetic Analysis of Interferon-α2b Containing DNL Molecules Administered as Intravenous Injections to Naïve Swiss-Webster Mice. | | | | | | | |
|---|---|---|---|---|---|---|---|
| Animal Number | IFN Dose (pmol) | Cₘₐₓ (pM) | T_{½α} (hours) | T_{½β} (hours) | AUC_{0.08→∞} (h*pM) | Elimination Rate (1/h) | MRT _{0.08→∞} (h) |
| *Recombinant Human Interferon-*α*2a* | | | | | | | |
| Animal No. 1 | 160 | 16,411 | 0.29 | 10.53 | 7,011 | 2.34 | 0.63 |
| Animal No. 2 | 160 | 21,835 | 0.31 | 7.14 | 10,147 | 2.15 | 0.78 |
| Mean | 160 | 19,123 | 0.30 | 8.84 | 8,579 | 2.25 | 0.71 |
| PEG-INTRON | | | | | | | |
| Animal No. 1 | 160 | 87,090 | 0.53 | 6.29 | 137,790 | 0.63 | 5.42 |
| Animal No. 2 | 160 | 105,774 | 0.43 | 5.11 | 150,905 | 0.70 | 4.79 |
| Mean | 160 | 96,432 | 0.48 | 5.70 | 144,348 | 0.67 | 5.11 |
| *IFN-*α*2b-IMP362* | | | | | | | |
| Animal No. 1 | 320 | 60,833 | 1.72 | 7.54 | 379,462 | 0.16 | 9.03 |
| Animal No. 2 | 320 | 97,089 | 1.43 | 10.14 | 570,336 | 0.17 | 11.56 |
| Mean | 320 | 78,961 | 1.58 | 8.84 | 474,899 | 0.17 | 10.30 |
| *IFN-*α*2b-IMP413* | | | | | | | |
| Animal No. 1 | 320 | 152,923 | 0.69 | 12.85 | 1,012,470 | 0.15 | 16.75 |
| Animal No. 2 | 320 | 100,495 | 4.03 | 28.53 | 1,179,056 | 0.09 | 26.56 |
| Mean | 320 | 126,709 | 2.36 | 20.69 | 1,095,763 | 0.12 | 21.66 |

### Anti-tumor therapeutic efficacy

An initial in vivo tumor therapy study demonstrated that the DNL-PEGylated interferons were more potent and longer-lasting compared to PEGINTRON™. Eight-week-old female C.B.-17 SCID mice were injected i.v. with a human Burkitt's lymphoma cell-line (Daudi) at 1.5 x 10⁷ cells per animal. There were 10 different treatment groups of 5 mice each. Equivalent units of activity of PEGINTRON™ , α2b-362 and α2b-413 were administered once every 7 days *via* s.c. injection in either the left or right flank at three different doses (3500, 7000, and 14000 Units). Therapy commenced 1 day after the Daudi cells were transplanted.

Mice were observed daily for signs of distress and paralysis. They were weighed weekly. In the event a mouse or mice lost greater than 15% of its body weight (but <20%) it was weighed every 2 days until it either gained back its weight to <15% loss or was sacrificed due to >20% loss. Mice were also terminated when hind-limb paralysis developed or if they became otherwise moribund.

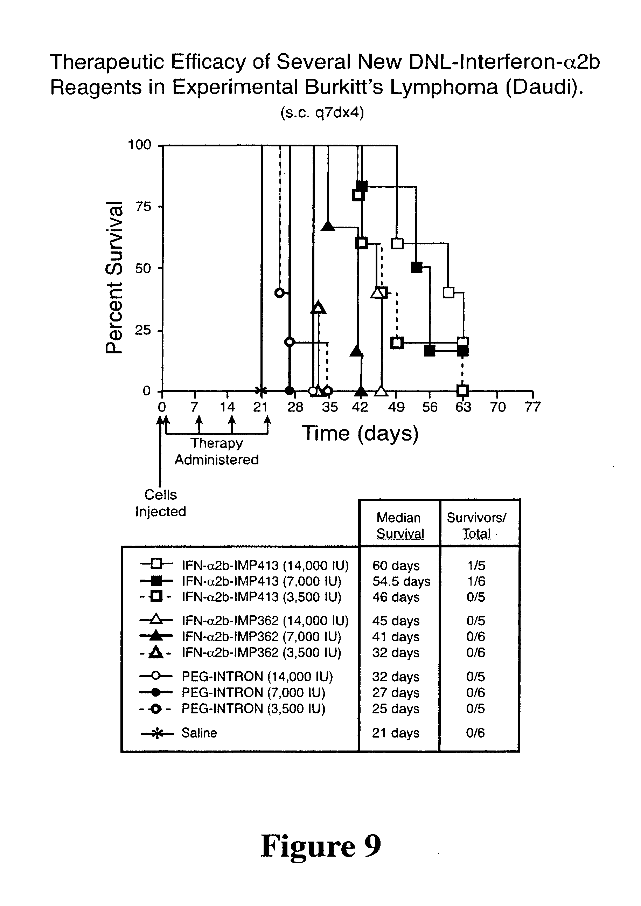

Survival curves generated from this study are shown in FIG. 9. PEGINTRON™, α2b-362, and α2b-413 all demonstrated significant improvement in survival when compared to saline control mice (*P*<0.0016). Except for the 3,500 IU dose of α2b-362, both α2b-413 and α2b-362 were superior to PEGINTRON™ when administered at equal activity doses (*P*≤0.0027). α2b-362 showed more than twice the potency of PEGINTRON™. Doses of 7,000 IU and 3,500 IU of α2b-362 were superior to 14,000 IU (*P*=0.0016) and 7,000 IU (*P*=0.0027) doses of PEGINTRON™, respectively. α2b-413 is more than four times as potent as PEGINTRON™ since a 3,500 IU dose of the former was superior to 14,000 IU of the latter (*P*=0.0027). α2b-413 was significantly better than α2b-362 (*P*<0.0025) when administered at equivalent doses. However, there were no statistically significant differences among the three doses of α2b-413, even though the 14,000 IU dose resulted in a median survival of 60 days in comparison to the 3,500 IU dose and its 46-day median survival (*P*=0.1255). The *in vivo* efficacy observed for α2b-362, α2b-413, and PEGINTRON™ thus correlate well with the PK data.

The increased bioavailability of α2b-362 and α2b-413 demonstrated by PK analysis contributes to the enhanced *in vivo* anti-tumor potency of DNL-PEGylated IFNα. In turn, these two factors allow for a less frequent dosing schedule used in tumor therapy. This was demonstrated with a similar *in vivo* tumor therapy study as above, in which equal units of activity of PEGINTRON™ or α2b-413 were administered with varied dosing schedules. This study was performed in 8-week-old female SCID mice injected i.v. with Daudi 1.5 x 10⁷ cells. There were 7 different treatment groups of 6-7 mice each. Each reagent (test and control) was administered 14,000 IU *via* a s.c. injection in either the left or right flank. Therapy was commenced 1 day after the Daudi-cells were administered to the mice. One set of mice was dosed once a week for 4 weeks (q7dx4), another dosed on a bi-weekly schedule over 8 weeks (q2wkx4), while the third set of mice was dosed once every 3 weeks over 12 weeks (q3wkx4). All the mice received a total of 4 injections.

Survival curves generated from this study are shown in FIG. 10. All animals that received either form of interferon at any of the various schedules had significantly improved survival in comparison to saline control mice (*P*<0.0009). Importantly, all the IFN-IMP413-treated mice had significantly improved survival when compared to those animals treated at the same schedule with PEGINTRON™ (*P*<0.0097). Of note, those mice treated every other week with IFN-IMP413 (q2wkx4) not only had significantly improved survival in comparison to those treated with PEGINTRON™ at the same schedule (MST= >54 days *versus* 28 days, respectively; P=0.0002), but were also significantly better than those animals treated weekly (q7dx4) with PEGINTRON™ (MST=36.5 days; *P*=0.0049). Further, survival of mice treated every three weeks with IFN-IMP413 (q3wkx4) was significantly better than those treated with PEGINTRON™ every two weeks (MST= 54 days *versus* 28 days; *P*=0.002) and approaches significance when compared to those treated weekly with PEGINTRON™ (*P*=0.0598).

These studies demonstrated that DNL-PEGylation of IFNα2b resulted in improved and long-lasting efficacy, even when compared with other PEGylated forms of IFNα2b, allowing for less frequent dosing. Similar enhancements are realized when this technology is applied to other cytokines (such as G-CSF and EPO), growth factors, enzymes, antibodies, immunomodulators, hormones, peptides, drugs, interference RNA, oligonucleotides, vaccines and other biologically active agents.

### Example 6. Generation of DDD module based on Granulocyte-Colony Stimulating Factor (G-CSF)

### Construction of G-CSF-DDD2-pdHL2 for expression in mammalian cells

The cDNA sequence for G-CSF was amplified by PCR resulting in sequences comprising the following features, in which XbaI and BamHI are restriction sites, the signal peptide is native to human G-CSF, and 6 His is a hexahistidine tag: XbaI---Signal peptideG-CSF ---6 His---BamHI. The resulting secreted protein consisted of G-CSF fused at its C-terminus to a polypeptide consisting of SEQ ID NO:5.

PCR amplification was accomplished using a full-length human G-CSF cDNA clone (Invitrogen IMAGE human cat# 97002RG Clone ID 5759022) as a template and the following oligonucleotides as primers:
G-CSF XbaI Left
   5'-TCTAGACACAGGACCTCATCATGGCTGGACCTGCCACCCAG-3' (SEQ ID NO:5)
G-CSF BamHI-Right
   5'-GGATCCATGATGGTGATGATGGTGTGACTTGGGCTGGGCAAGGTGGCGTAG-3' (SEQ ID NO:6)

The PCR amplimer was cloned into the pGemT vector. A DDD2-pdHL2 mammalian expression vector was prepared for ligation with G-CSF by digestion with XbaI and Bam HI restriction endonucleases. The G-CSF amplimer was excised from pGemT with XbaI and Bam HI and ligated into the DDD2-pdHL2 vector to generate the expression vector G-CSF-DDD2-pdHL2.

### Mammalian expression of G-CSF-DDD2

G-CSF-pdHL2 was linearized by digestion with SalI enzyme and stably transfected into Sp/EEE myeloma cells by electroporation. Clones were selected with media containing 0.15 µM MTX. Clone # 4 was shown to produce 0.15 mg/L of G-CSF-DDD2 by sandwich ELISA.

### Purification of G-CSF-DDD2 from batch cultures grown in roller bottles

Approximately 3 mg of G-CSF-DDD2 is purified as descried in Example 2. Clone 4 is expanded to 34 roller bottles containing a total of 20 L of Hybridoma SFM with 0.4 µM MTX and allowed to reach terminal culture. The supernatant fluid is clarified by centrifugation, filtered (0.2 µM), diafiltered into 1X Binding buffer (10 mM Imidazole, 0.5 M NaCl, 50 mM NaH₂PO₄, pH 7.5 and concentrated. The concentrate is loaded onto a Ni-NTA column, which is washed with 0.02% Tween 20 in 1X binding buffer and then 30 mM imidazole, 0.02% Tween 20, 0.5 M NaCl, 50 mM NaH₂PO₄, pH 7.5. The product is eluted with 250 mM imidazole, 0.02% Tween 20, 150 mM NaCl, 50 mM NaH₂PO₄, pH 7.5.

### Example 7. Generation of PEGylated G-CSF by DNL

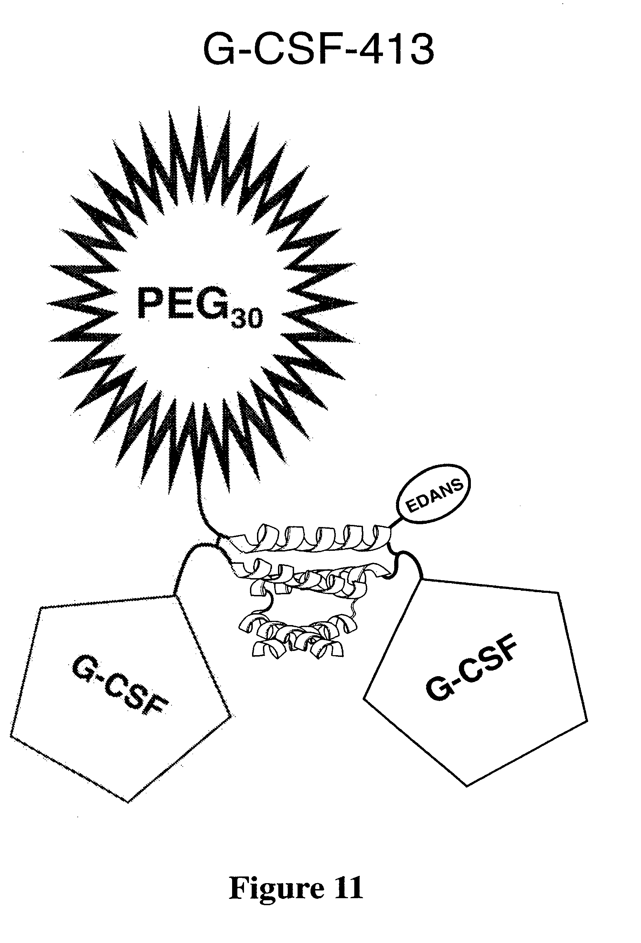

A cartoon drawing depicting the structure of G-CSF-413 having two copies of G-CSF coupled to a 30 kDa PEG is provided in FIG. 11. A DNL reaction is performed by the addition of reduced and lyophilized IMP413 in 10-fold molar excess to G-CSF-DDD2 in PBS. After 6 h at room temperature in the dark, the reaction mixture is purified by immobilized metal affinity chromatography using Ni-NTA.

### Example 8. Generation of DDD module based on Erythropoeitin (EPO)

### Construction of G-CSF-DDD2-pdHL2 for expression in mammalian cells

The cDNA sequence for EPO was amplified by PCR resulting in sequences comprising the following features, in which XbaI and BamHI are restriction sites, the signal peptide is native to human EPO, and 6 His is a hexahistidine tag: XbaI---Signal peptide-EPO ---6 His---BamHI. The resulting secreted protein consists of EPO fused at its C-terminus to a polypeptide consisting of SEQ ID NO:2.

PCR amplification was accomplished using a full-length human EPO cDNA clone as a template and the following oligonucleotides as primers:
EPO Xba I left
   5'-TCTAGACACAGGACCTCATCATGGGGGTGCACGAATGTCC-3' (SEQ ID NO:7)
EPO BamHI Right
   5'-GGATCCATGATGGTGATGATGGTGTGACTTTCTGTCCCCTGTCCTGCAG-3' (SEQ ID NO:8)

The PCR amplimer was cloned into the pGemT vector. A DDD2-pdHL2 mammalian expression vector was prepared for ligation with EPO by digestion with XbaI and Bam HI restriction endonucleases. The EPO amplimer was excised from pGemT with XbaI and Bam HI and ligated into the DDD2-pdHL2 vector to generate the expression vector EPO-DDD2-pdHL2.

### Mammalian expression of EPO-DDD2

EPO-pdHL2 was linearized by digestion with SalI enzyme and stably transfected into Sp/EEE myeloma cells by electroporation. Clones were selected with media containing 0.15 µM MTX. Clones # 41, 49 and 37 each were shown to produce ~0.5 mg/L of EPO by an ELISA using Nunc Immobilizer Nickel-Chelate plates to capture the His-tagged fusion protein and detection with anti-EPO antibody.

### Purification of EPO from batch cultures grown in roller bottles

Approximately 2.5 mg of EPO-DDD2 was purified by IMAC from 9.6 liters of serum-free roller bottle culture as described in Example 2. SDS-PAGE and immunoblot analysis indicated that the purified product constituted approximately 10% of the total protein following IMAC (not shown). Under reducing conditions the EPO-DDD2 polypeptide was resolved as a broad band with a Mᵣ (40 - 45 kDa) greater than its calculated mass (28 kDa) due to extensive and heterogeneous glycosylation. Under non-reducing conditions the EPO-DDD2 primarily resolved as a disulfide-linked covalent dimer (mediated by DDD2) with a Mᵣ of 80-90 kDa.

### Example 9. DNL conjugation of EPO-DDD2 with a Fab-AD2 module

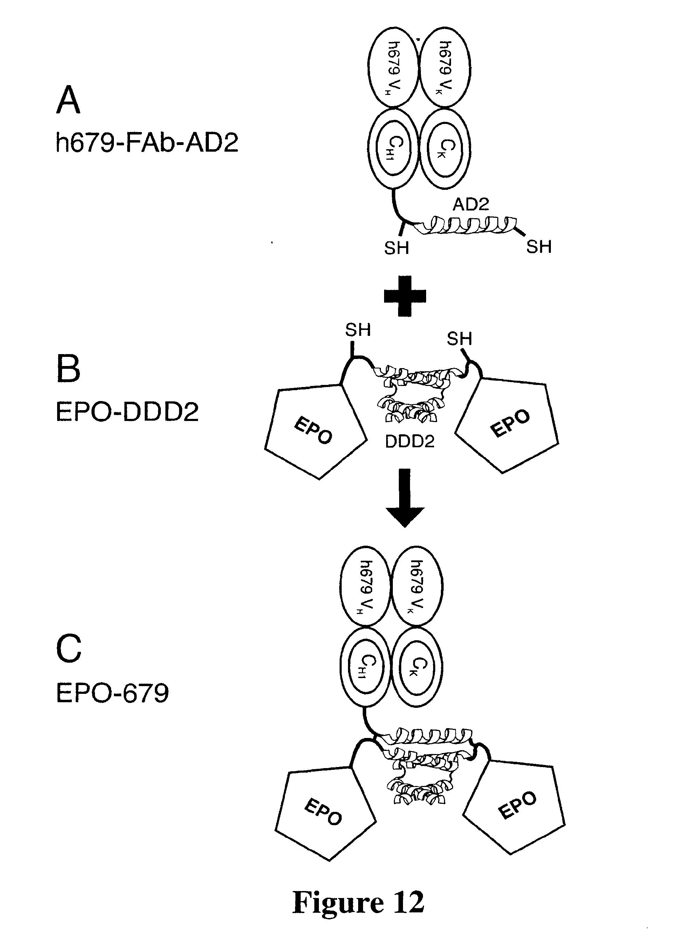

h679 is a humanized monoclonal antibody that is highly specific for the hapten HSG (histamine-succinyl-glycine). Production of an h679-Fab-AD2 module, which is depicted in the cartoon drawing in FIG. 12A, has been described previously (Rossi et.al, Proc. Natl. Acad. Sci. USA. 2006; 103:6841). A cartoon drawing depicting the dimeric structure of EPO-DDD2 is provided in FIG. 12B. A small-scale preparation of EPO-679 (EPO-DDD2 x h679-Fab-AD2) was made by DNL. EPO-DDD2 (1 mg) was reacted overnight with h679-Fab-AD2 (1 mg) in PBS containing 1mM reduced glutathione and 2 mM oxidized glutathione. The DNL conjugate was purified by HSG-based affinity chromatography as described previously (Rossi et. al, Proc. Natl. Acad. Sci. USA. 2006; 103:6841). A cartoon drawing depicting the structure of EPO-679 with two EPO moieties and h679-Fab is provided in FIG. 12C. Coomassie blue staining of SDS-PAGE gels demonstrated the creation of EP-679 (not shown). The DNL product, which resolved as a broad band with a Mᵣ of 150 - 170 kDa under non-reducing conditions, was highly purified and consisted only of the three constituent polypeptides (EPO, h679-Fd-AD2 and h679 Kappa) as demonstrated by SDS-PAGE under reducing conditions (not shown).

### Example 10. Biological activity of EPO-DDD2 and EPO-679

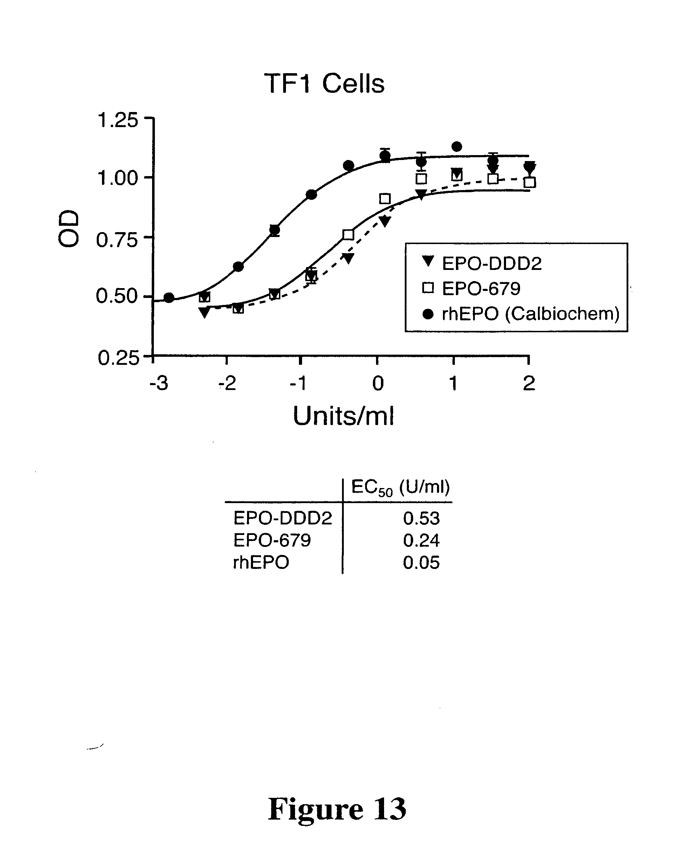

EPO-DDD2 and EPO-679 were assayed for their ability to stimulate the growth of EPO-responsive TF1 cells (ATCC) using recombinant human EPO (Calbiochem) as a positive control. TF1 cells were grown in RPMI 1640 media supplemented with 20% FBS without GM-CSF supplementation in 96-well plates containing 1 x 10⁴ cells/well. The concentrations (units/ml) of the EPO constructs were determined using a commercial kit (Human erythropoietin ELISA kit, Stem Cell Research, Cat# 01630). Cells were cultured in the presence of rhEPO, EPO-DDD2 or EPO-679 at concentrations ranging from 900 U/ml to 0.001 U/ml for 72 hours. The viable cell densities were compared by MTS assay using 20 µl of MTS reagent/well incubated for 6 hours before measuring the OD490 in a 96-well plate reader. Dose response curves and EC50 values were generated using Graph Pad Prism software (FIG. 13). Both EPO-DDD2 and EPO-679 showed *in vitro* biological activity at approximately 10% of the potency of rhEPO.

### Example 11. Generation of PEGylated EPO by DNL

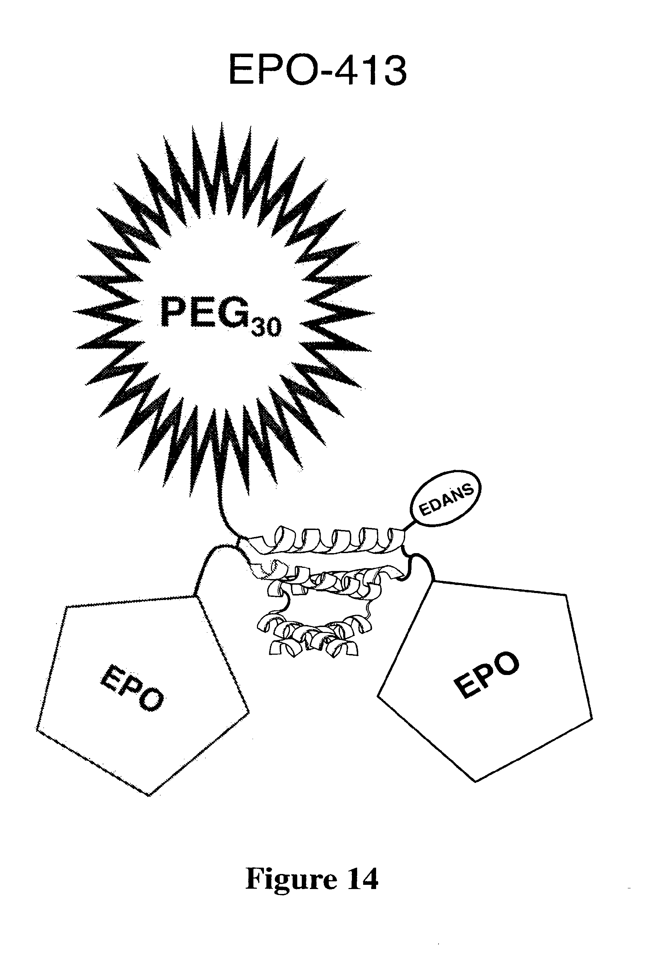

A cartoon drawing depicting the structure of EPO-413 having two copies of EPO coupled to a 30 kDa PEG is provided in FIG. 14. A DNL reaction is performed by the addition of reduced and lyophilized IMP413 in 10-fold molar excess to EPO-DDD2 in PBS. After 6 h at room temperature in the dark, the reaction mixture is purified by immobilized metal affinity chromatography using Ni-NTA.

### Example 12. Production of 2-PEG:1-Effector moiety Complexes

In alternative embodiments, it is desirable to make PEGylated complexes with a stoichiometry of 2 PEG moieties to 1 effector moiety. Such PEGylated complexes are readily made by the methods of Examples 1-3 above, by attaching the PEG moiety to the DDD sequence and the active agent to the AD sequence. A PEGylated complex with a 2:1 stoichiometry of PEG to IFN-α2b is prepared by a modification of the methods of Examples 1-3. The complex exhibits stability in serum and shows interferon activity that is lower than the PEGylated complex with a 1:2 stoichiometry of PEG to IFN-α2b. However, clearance rate for the bi-PEGylated complex is slower than the clearance rate for the mono-PEGylated complex.

### Example 13. Production of Antibody Fragments For PEGylation

Fab antibody fragments may be produced as fusion proteins containing either a DDD or AD sequence. Independent transgenic cell lines are developed for each Fab fusion protein. Once produced, the modules can be purified if desired or maintained in the cell culture supernatant fluid. Following production, any (Fab-DDD)₂ module can be combined with any PEG-AD module, or any Fab-AD module can be combined with any (PEG-DDD)₂ module to generate a PEGylated Fab construct.
DDD1: SHIQIPPGLTELLQGYTVEVLRQQPPDLVEFAVEYFTRLREARA (SEQ ID NO:10)
DDD2: CGHIQIPPGLTELLQGYTVEVLRQQPPDLVEFAVEYFTRLREARA (SEQ ID NO:11)
AD1: QIEYLAKQIVDNAIQQA (SEQ ID NO:12)
AD2: CGQIEYLAKQIVDNAIQQAGC (SEQ ID NO: 1)

The plasmid vector pdHL2 has been used to produce a number of antibodies and antibody-based constructs. See Gillies et al., J Immunol Methods (1989), 125:191-202; Losman et al., Cancer (Phila) (1997), 80:2660-6. The di-cistronic mammalian expression vector directs the synthesis of the heavy and light chains of IgG. The vector sequences are mostly identical for many different IgG-pdHL2 constructs, with the only differences existing in the variable domain (VH and VL) sequences. Using molecular biology tools known to those skilled in the art, these IgG expression vectors can be converted into Fab-DDD or Fab-AD expression vectors. To generate Fab-DDD expression vectors, the coding sequences for the hinge, CH2 and CH3 domains of the heavy chain are replaced with a sequence encoding the first 4 residues of the hinge, a 14 residue Gly-Ser linker and the first 44 residues of human RIIα (referred to as DDD1). To generate Fab-AD expression vectors, the sequences for the hinge, CH2 and CH3 domains of IgG are replaced with a sequence encoding the first 4 residues of the hinge, a 15 residue Gly-Ser linker and a 17 residue synthetic AD called AKAP-*IS* (referred to as AD1), which was generated using bioinformatics and peptide array technology and shown to bind RIIα dimers with a very high affinity (0.4 nM). See Alto, et al. Proc. Natl. Acad. Sci., U.S.A (2003), 100:4445-50.

Two shuttle vectors were designed to facilitate the conversion of IgG-pdHL2 vectors to either Fab-DDD1 or Fab-AD1 expression vectors, as described below.

### Preparation of CH1

The CH1 domain was amplified by PCR using the pdHL2 plasmid vector as a template. The left PCR primer consists of the upstream (5') of the CH1 domain and a SacII restriction endonuclease site, which is 5' of the CH1 coding sequence. The right primer consists of the sequence coding for the first 4 residues of the hinge (PKSC) followed by GGGGS with the final two codons (GS) comprising a Bam HI restriction site.
*5' of CH1 Left Primer*
5'GAACCTCGCGGACAGTTAAG-3' (SEQ ID NO:13)
*CH1*+*G₄S-Bam Right*
5'GGATCCTCCGCCGCCGCAGCTCTTAGGTTTCTTGTCCACCTTGGTGTTGCTGG-3' (SEQ ID NO:14)

The 410 bp PCR amplimer was cloned into the pGemT PCR cloning vector (Promega, Inc.) and clones were screened for inserts in the T7 (5') orientation.

### Construction of (G₄S)₂DDD1

A duplex oligonucleotide, designated (G₄S)₂DDD1, was synthesized by Sigma Genosys (Haverhill, UK) to code for the amino acid sequence of DDD1 preceded by 11 residues of the linker peptide, with the first two codons comprising a BamHI restriction site. A stop codon and an EagI restriction site are appended to the 3'end. The encoded polypeptide sequence is shown below.
GSGGGGSGGGGSHIQIPPGLTELLQGYTVEVLRQQPPDLVEFAVEYFTRLREARA (SEQ ID NO:15)

The two oligonucleotides, designated RIIA1-44 top and RIIA1-44 bottom, that overlap by 30 base pairs on their 3' ends, were synthesized (Sigma Genosys) and combined to comprise the central 154 base pairs of the 174 bp DDD1 sequence. The oligonucleotides were annealed and subjected to a primer extension reaction with Taq polymerase.
*RIIA1-44 top* *RIIA1-44 bottom*

Following primer extension, the duplex was amplified by PCR using the following primers:
*G4S Bam-Left*
   5'-GGATCCGGAGGTGGCGGGTCTGGCGGAGGT-3' (SEQ ID NO:18)
*1-44 stop Eag Right*
   5'-CGGCCGTCAAGCGCGAGCTTCTCTCAGGCG-3' (SEQ ID NO:19)

This amplimer was cloned into pGemT and screened for inserts in the T7 (5') orientation.

### Construction of (G₄S)₂-AD1

A duplex oligonucleotide, designated (G₄S)₂-AD1, was synthesized (Sigma Genosys) to code for the amino acid sequence of AD1 preceded by 11 residues of the linker peptide with the first two codons comprising a BamHI restriction site. A stop codon and an EagI restriction site are appended to the 3'end. The encoded polypeptide sequence is shown below.

### GSGGGGSGGGGSQIEYLAKQIVDNAIQQA (SEQ ID NO:20)

Two complimentary overlapping oligonucleotides, designated AKAP-IS Top and AKAP-IS Bottom, were synthesized.
*AKAP-IS Top* *AKAP-IS Bottom*

The duplex was amplified by PCR using the following primers:
*G4S Bam-Left*
   5'-GGATCCGGAGGTGGCGGGTCTGGCGGAGGT-3' (SEQ ID NO:23)
*AKAP-IS stop Eag Right*
   5'-CGGCCGTCAGGCCTGCTGGATG-3' (SEQ ID NO:24)

This amplimer was cloned into the pGemT vector and screened for inserts in the T7 (5') orientation.

### Ligating DDD1 with CH1

A 190 bp fragment encoding the DDD1 sequence was excised from pGemT with BamHI and NotI restriction enzymes and then ligated into the same sites in CH1-pGemT to generate the shuttle vector CH1-DDD1-pGemT.

### Ligating AD1 with CH1

A 110 bp fragment containing the AD1 sequence was excised from pGemT with BamHI and NotI and then ligated into the same sites in CH1-pGemT to generate the shuttle vector CH1-AD1-pGemT.

### Cloning CH1-DDD1 or CH1-AD1 into pdHL2-based vectors

With this modular design either CH1-DDD1 or CH1-AD1 can be incorporated into any IgG construct in the pdHL2 vector. The entire heavy chain constant domain is replaced with one of the above constructs by removing the SacII/EagI restriction fragment (CH1-CH3) from pdHL2 and replacing it with the SacII/EagI fragment of CH1-DDD1 or CH1-AD1, which is excised from the respective pGemT shuttle vector.

### Construction of C-DDD1-Fd-hMN-14-pdHL2

The hMN-14 antibody is a humanized CEA-binding antibody comprising the murine MN-14 CDR sequences (see, e.g., U.S. Patent No. 6,676,924). C-DDD1-Fd-hMN-14-pdHL2 is an expression vector for production of a stable dimer that comprises two copies of a fusion protein C-DDD1-Fab-hMN-14, in which DDD1 is linked to hMN-14 Fab at the carboxyl terminus of CH1 via a flexible peptide spacer. The plasmid vector hMN14(I)-pdHL2, which has been used to produce hMN-14 IgG, was converted to C-DDD1-Fd-hMN-14-pdHL2 by digestion with SacII and EagI restriction endonucleases to remove the CH1-CH3 domains and insertion of the CH1-DDD1 fragment, which was excised from the CH1-DDD1-SV3 shuttle vector with SacII and EagI.

### C-DDD2-Fd-hMN-14-pdHL2

C-DDD2-Fd-hMN-14-pdHL2 is an expression vector for production of C-DDD2-Fab-hMN-14, which possesses a dimerization and docking domain sequence of DDD2 appended to the carboxyl terminus of the Fd via a 14 amino acid residue Gly/Ser peptide linker. The fusion protein secreted is composed of two identical copies of hMN-14 Fab held together by non-covalent interaction of the DDD2 domains.

The expression vector was engineered as follows. Two overlapping, complimentary oligonucleotides, which comprise the coding sequence for part of the linker peptide (GGGGSGGGCG, SEQ ID NO:25) and residues 1-13 of DDD2, were made synthetically. The oligonucleotides were annealed and phosphorylated with T4 PNK, resulting in overhangs on the 5' and 3' ends that are compatible for ligation with DNA digested with the restriction endonucleases BamHI and PstI, respectively.
*G4S-DDD2 top* *G4S-DDD2 bottom*

The duplex DNA was ligated with the shuttle vector CH1-DDD1-pGemT, which was prepared by digestion with BamHI and PstI, to generate the shuttle vector CH1-DDD2-pGemT. A 507 bp fragment was excised from CH1-DDD2-pGemT with SacII and EagI and ligated with the IgG expression vector hMN14(I)-pdHL2, which was prepared by digestion with SacII and EagI. The final expression construct is C-DDD2-Fd-hMN-14-pdHL2.

### Example 14. PEGylation of Fab Antibody Fragments

The C-DDD2-Fd-hMN14-pdHL2 vector is transfected into Sp/EEE cells (see U.S. Patent Application Serial No. 11/487,215) and used to produce C-DDD2-Fab-hMN-14. The di-cistronic expression vector directs the synthesis and secretion of both hMN-14 kappa light chain and C-DDD2-Fd-hMN-14, which combine to form C-DDD2-Fab-hMN14. The C-DDD2-Fab-hMN-14 spontaneously forms dimers, which are mixed with an equimolar amount of IMP362, IMP413 or IMP457, resulting in the production of a PEGylated C-DDD2-Fab-hMN-14 dimer. The hMN-14 Fab moiety retains its binding specificity for the CEA antigen. Injection into nude mice bearing a CEA-expressing tumor shows that the PEGylated C-DDD2-Fab-hMN-14 exhibits a significantly prolonged circulating half-life compared to non-PEGylated hMN-14 F(ab)₂, resulting in improved efficacy with a less frequent dosing schedule.

### Example 15. Creation of C-H-AD2-IgG-pdHL2 expression vectors.

The pdHL2 mammalian expression vector has been used to mediate the expression of many recombinant IgGs. A plasmid shuttle vector was produced to facilitate the conversion of any IgG-pdHL2 vector into a C-H-AD2-IgG-pdHL2 vector. The gene for the Fc (CH2 and CH3 domains) was amplified using the pdHL2 vector as a template and the oligonucleotides *Fc BghlII Left* and *Fc Bam*-*EcoRI Right* as primers.
*Fc BglII Left*
5'-AGATCTGGCGCACCTGAACTCCTG-3' (SEQ ID NO:28)
*Fc Bam-EcoRI Right*
5'-GAATTCGGATCCTTTACCCGGAGACAGGGAGAG-3' (SEQ ID NO:29)

The amplimer was cloned in the pGemT PCR cloning vector. The Fc insert fragment was excised from pGemT and ligated with AD2-pdHL2 vector to generate the shuttle vector Fc-AD2-pdHL2.

To convert any IgG-pdHL2 expression vector to a C-H-AD2-IgG-pdHL2 expression vector, an 861 bp BsrGI / NdeI restriction fragment is excised from the former and replaced with a 952 bp BsrGI / NdeI restriction fragment excised from the Fc-AD2-pdHL2 vector. BsrGI cuts in the CH3 domain and NdeI cuts downstream (3') of the expression cassette.

### Example 16. Production of C-H-AD2-hLL2 IgG

Epratuzumab, or hLL2 IgG, is a humanized anti-human CD22 MAb (see, e.g., U.S. Patent Nos. 5,443,953; 5,789,554; 6,187,287; 7,074,403). An expression vector for C-HAD2-hLL2 IgG was generated from hLL2 IgG-pdHL2, as described in Example 15, and used to transfect Sp2/0 myeloma cells by electroporation. Following transfection, the cells were plated in 96-well plates and transgenic clones were selected in media containing methotrexate. Clones were screened for C-H-AD2-hLL2 IgG productivity by a sandwich ELISA using 96-well microtitre plates coated with an hLL2-specific anti-idiotype MAb and detection with peroxidase-conjugated anti-human IgG. Clones were expanded to roller bottles for protein production and C-H-AD2-hLL2 IgG was purified from the spent culture media in a single step using Protein-A affinity chromatography. SE-HPLC analysis resolved two protein peaks (not shown). The retention time of the slower eluted peak (8.63 min) is similar to hLL2 IgG. The retention time of the faster eluted peak (7.75 min) is consistent with a ~300 kDa protein. It was later determined that this peak represents disulfide linked dimers of C-HAD2-hLL2-IgG. This dimer is reduced to the monomeric form during the DNL reaction. SDS-PAGE analysis demonstrated that the purified C-H-AD2-hLL2-IgG consisted of both monomeric and disulfide-linked dimeric forms of the module (not shown). Protein bands representing these two forms are evident by SDS-PAGE under non-reducing conditions, while under reducing conditions all of the forms are reduced to two bands representing the constituent polypeptides (Heavy chain-AD2 and kappa chain). No other contaminating bands were detected.

### Example 17. Production of C-H-AD2-hA20 IgG

hA20 IgG is a humanized anti-human CD20 MAb (see, e.g., U.S. Patent No. 7,154,164). An expression vector for C-H-AD2-hA20 IgG was generated from hA20 IgG-pDHL2, as described in Example 15, and used to transfect Sp2/0 myeloma cells by electroporation. Following transfection, the cells were plated in 96-well plates and transgenic clones were selected in media containing methotrexate. Clones were screened for C-H-AD2-hA20 IgG productivity by a sandwich ELISA using 96-well microtitre plates coated with an hA20-specific anti-idiotype MAb and detection with peroxidase-conjugated anti-human IgG. Clones were expanded to roller bottles for protein production and C-H-AD2-hA20 IgG was purified from the spent culture media in a single step using Protein-A affinity chromatography. SE-HPLC and SDS-PAGE analyses gave very similar results to those obtained for C-H-AD2-hLL2 IgG in Example 16.

### Example 18. Production of PEGylated IgG

The C-H-AD2-hLL2 IgG or C-H-AD2-hA20 IgG antibodies are mixed with PEG-DDD2 to form PEGylated hLL2 or hA20. The hLL2 and hA20 retain their binding specificity for the CD22 and CD20 antigens, respectively. Injection into nude mice bearing a CD20- or CD22-expressing lymphomas shows that the PEGylated antibodies exhibit a significantly prolonged circulating half-life compared to non-PEGylated antibody, resulting in improved efficacy with a less frequent dosing schedule.

### EMBODIMENTS

Embodiment 1: A PEGylated complex comprising:
   a) An effector moiety attached to a DDD (dimerization and docking domain) sequence; and
   b) a PEG moiety attached to an AD (anchor domain) sequence;
   wherein two DDD sequences bind to one AD sequence to form a PEGylated complex.
Embodiment 2: The complex of embodiment 1, further comprising disulfide bonds between the DDD and AD sequences.
Embodiment 3: The complex of embodiment 1, wherein the DDD sequence comprises the 44 N-terminal amino acids of PKA RIIa.
Embodiment 4: The complex of embodiment 1, wherein the DDD sequence comprises SEQ ID NO:2.
Embodiment 5: The complex of embodiment 1, wherein the PEG moiety is capped at one end with a methoxy group.
Embodiment 6: The complex of embodiment 1, wherein the effector moiety is selected from the group consisting of an enzyme, a cytokine, a chemokine, a growth factor, a peptide, an aptamer, hemoglobin, an antibody and an antibody fragment.
Embodiment 7: The complex of embodiment 6, wherein the effector moiety is selected from the group consisting of interferon-α, interferon-β, interferon-γ, MIF, HMGB-1 (high mobility group box protein 1), TNF-α, IL-1, IL-2, IL-3, IL-4, IL-5, IL-6, IL-7, IL-8, IL-9, IL-10, IL-11, IL- 12, IL-13, IL-15, IL-16, IL-17, IL-18, IL-19, IL-23, IL-24, CCL19, CCL21, MCP-I, RANTES, MIP-1A, MIP-1B, ENA-78, MCP-1, IP-10, Gro-β, Eotaxin, G-CSF, GM-CSF, SCF, PDGF, MSF, Flt-3 ligand, erythropoietin, thrombopoietin, hGH, CNTF, leptin, oncostatin M, VEGF, EGF, FGF, P1GF, insulin, hGH, calcitonin, Factor VIII, IGF, somatostatin, tissue plasminogen activator, and LIF.
Embodiment 8: The complex of embodiment 1, wherein the PEG moiety attached to an AD sequence comprises IMP350, IMP360, IMP362, IMP413 or IMP457.
Embodiment 9: The complex of embodiment 1, wherein the effector moiety is interferon (IFN)-α2b, G-CSF or erythropoietin.
Embodiment 10: The complex of embodiment 1, wherein the effector moiety attached to a DDD sequence is a fusion protein.
Embodiment 11: The complex of embodiment 1, wherein each complex comprises one PEG moiety and two effector moieties.
Embodiment 12: The complex of embodiment 1, wherein the clearance rate of the PEGylated complex from serum is at least an order of magnitude slower than the clearance rate of the unPEGylated effector moiety.
Embodiment 13: A PEGylated complex comprising:
   a) a effector moiety attached to an AD sequence; and
   b) a PEG moiety attached to a DDD sequence;
   wherein two DDD sequences bind to one AD sequence to form a PEGylated complex.
Embodiment 14: The complex of embodiment 13, wherein each complex comprises two PEG moieties and one effector moiety.
Embodiment 15: The complex of embodiment 13, wherein the clearance rate of the PEGylated complex from serum is at least an order of magnitude slower than the clearance rate of the unPEGylated effector moiety.
Embodiment 16: A method of PEGylating a effector moiety comprising:
   a) attaching an effector moiety to a DDD sequence;
   b) attaching a PEG moiety to an AD sequence; and
   c) allowing the DDD sequence to bind to the AD sequence to form a PEGylated complex comprising two effector moiety-DDD sequences and one PEG-AD sequence.
Embodiment 17: The method of embodiment 16, wherein the effector moiety is selected from the group consisting of an enzyme, a cytokine, a chemokine, a growth factor, a peptide, an aptamer, hemoglobin, an antibody and an antibody fragment.
Embodiment 18. The method of embodiment 16, wherein the clearance rate of the PEGylated complex from serum is at least an order of magnitude slower than the clearance rate of the unPEGylated effector moiety.
Embodiment 19: A method of PEGylating a effector moiety comprising:
   a) attaching a effector moiety to an AD sequence;
   b) attaching a PEG moiety to a DDD sequence; and
   c) allowing the DDD sequence to bind to the AD sequence to form a PEGylated complex comprising two effector moiety-DDD sequences and one PEG-AD sequence.
Embodiment 20: A method of treating a disease comprising:
   a) obtaining a PEGylated complex according to embodiment 1 , wherein the effector moiety is a therapeutic agent for the disease; and
   b) administering the PEGylated complex to a subject with the disease.
Embodiment 21: The method of embodiment 20, wherein the effector moiety is selected from the group consisting of MIF, HMGB-I (high mobility group box protein 1), TNF-α, IL-1, IL-2, IL- 3, IL-4, IL-5, IL-6, IL-7, IL-8, IL-9, IL-10, IL-11, IL-12, IL-13, IL-15, IL-16, IL-17, IL- 18, IL-19, IL-23, IL-24, CCL19, CCL21, IL-8, MCP-1, RANTES, MIP-1A, MIP-1B, ENA-78, MCP-1, IP-10, Gro-β, Eotaxin, interferon-α, -β, -λ, G-CSF, GM-CSF, SCF, PDGF, MSF, Flt-3 ligand, erythropoietin, thrombopoietin, hGH, CNTF, leptin, oncostatin M, VEGF, EGF, FGF, PIGF, insulin, hGH, calcitonin, Factor VIII, IGF, somatostatin, tissue plasminogen activator, and LIF.
Embodiment 22: The method of embodiment 20, wherein the disease is cancer, hyperplasia, diabetes, autoimmune disease, diabetic retinopathy, macular degeneration, inflammatory bowel disease, Crohn's disease, ulcerative colitis, rheumatoid arthritis, sarcoidosis, asthma, edema, pulmonary hypertension, psoriasis, corneal graft rejection, neovascular glaucoma, Osler- Webber Syndrome, myocardial angiogenesis, plaque neovascularization, restenosis, neointima formation after vascular trauma, telangiectasia, hemophiliac joints, angiofibroma, fibrosis associated with chronic inflammation, lung fibrosis, deep venous thrombosis or wound granulation.
Embodiment 23: The method of embodiment 20, wherein the autoimmune disease is acute idiopathic thrombocytopenic purpura, chronic idiopathic thrombocytopenic purpura, dermatomyositis, Sydenham's chorea, myasthenia gravis, systemic lupus erythematosus, lupus nephritis, rheumatic fever, polyglandular syndromes, bullous pemphigoid, juvenile diabetes mellitus, Henoch-Schonlein purpura, post-streptococcal nephritis, erythema nodosum, Takayasu's arteritis, Addison's disease, rheumatoid arthritis, multiple sclerosis, sarcoidosis, ulcerative colitis, erythema multiforme, IgA nephropathy, polyarteritis nodosa, ankylosing spondylitis, Goodpasture's syndrome, thromboangitis obliterans, Sjogren's syndrome, primary biliary cirrhosis, Hashimoto's thyroiditis, thyrotoxicosis, scleroderma, chronic active hepatitis, polymyositis/dermatomyositis, polychondritis, pemphigus vulgaris, Wegener's granulomatosis, membranous nephropathy, amyotrophic lateral sclerosis, tabes dorsalis, giant cell arteritis/polymyalgia, pernicious anemia, rapidly progressive glomerulonephritis, psoriasis or fibrosing alveolitis.
Embodiment 24: The method of embodiment 22, wherein the cancer is selected from the group consisting of acute lymphoblastic leukemia, acute myelogenous leukemia, biliary cancer, breast cancer, bone cancer, cervical cancer, chronic lymphocytic leukemia, chronic myelogenous leukemia, colorectal cancer, endometrial cancer, esophageal, gastric, head and neck cancer, Hodgkin's lymphoma, lung cancer, medullary thyroid cancer, non-Hodgkin's lymphoma, multiple myeloma, renal cancer, ovarian cancer, pancreatic cancer, glioma, melanoma, liver cancer, prostate cancer, and urinary bladder cancer.
Embodiment 25: The method of embodiment 20, wherein the PEGylated complex has only one PEG moiety attached to each PEGylated complex.

## Claims

1. A PEGylated complex comprising:
a) an effector moiety attached to a dimerization and docking domain (DDD) sequence of protein kinase A (PKA) RIIα, wherein the effector moiety is an interferon (IFN)-α2b ; and
b) a PEG moiety attached to an anchor domain (AD) sequence of an A-kinase anchoring protein (AKAP);
wherein two DDD sequences bind to one AD sequence to form a PEGylated complex.

2. The complex of claim 1, further comprising disulfide bonds between the DDD and AD sequences.

3. The complex of claim 1, wherein the DDD sequence comprises the 44 N-terminal amino acids of PKA RIIa.

4. The complex of claim 1, wherein the DDD sequence comprises SEQ ID NO:2.

5. The complex of claim 1, wherein the PEG moiety is capped at one end with a methoxy group.

6. The complex of claim 1, wherein the PEG moiety attached to an AD sequence comprises IMP350, IMP360, IMP362, IMP413 or IMP457,
wherein
IMP350 is CGQIEYLAKQIVDNAIQQAGC(SS-tbu)-NH₂ (SEQ ID NO:1),
IMP360 is CGQIEYLAKQIVDNAIQQAGC(SS-tbu)G-EDANS (SEQ ID NO:1),
IMP362 is PEG₂₀- CGQIEYLAKQIVDNAIQQAGC(SS-tbu)G-EDANS,
IMP413 is PEG₃₀- CGQIEYLAKQIVDNAIQQAGC(SS-tbu)G-EDANS, and
IMP457 is the reaction product of Ac-C-PEG₃-C(S-*t*Bu)GQIEYLAKQIVDNAIQQAGC(S-*t*Bu)G-NH₂ and mPEG2-MAL-40K of Fig. 16 reacted as described in Example 1.

7. The complex of claim 1, wherein the clearance rate of the PEGylated complex from serum is at least an order of magnitude slower than the clearance rate of the unPEGylated effector moiety.

8. A PEGylated complex comprising:
a) an effector moiety attached to an anchor domain (AD) sequence of an A-kinase anchoring protein (AKAP), wherein the effector moiety is an interferon (IFN)-α2b ; and
b) a PEG moiety attached to a dimerization and docking domain (DDD) sequence of protein kinase A (PKA) RIIα;
wherein two DDD sequences bind to one AD sequence to form a PEGylated complex.

9. The complex of claim 8, wherein the clearance rate of the PEGylated complex from serum is at least an order of magnitude slower than the clearance rate of the unPEGylated effector moiety.

10. A method of PEGylating an effector moiety comprising:
a) attaching an effector moiety to a dimerization and docking domain (DDD) sequence of protein kinase A (PKA) RIIα, wherein the effector moiety is an interferon (IFN)-α2b ;
b) attaching a PEG moiety to an anchor domain (AD) sequence of an A-kinase anchoring protein (AKAP); and
c) allowing the DDD sequence to bind to the AD sequence to form a PEGylated complex comprising two effector moiety-DDD sequences and one PEG-AD sequence.

11. The method of claim 10, wherein the clearance rate of the PEGylated complex from serum is at least an order of magnitude slower than the clearance rate of the unPEGylated effector moiety.

12. A method of PEGylating an effector moiety comprising:
a) attaching a effector moiety to an anchor domain (AD) sequence of an A-kinase anchoring protein (AKAP), wherein the effector moiety is aninterferon (IFN)-α2b ;
b) attaching a PEG moiety to a dimerization and docking domain (DDD) sequence of protein kinase A (PKA) RIIα; and
c) allowing the DDD sequence to bind to the AD sequence to form a PEGylated complex comprising two effector moiety-DDD sequences and one PEG-AD sequence.

## Patentansprüche

1. PEGylierter Komplex umfassend:
a) einen Effektoranteil, der an eine Sequenz einer Dimerisierungs- und Kopplungsdomäne (DDD) von Proteinkinase A (PKA) RIIα gebunden ist, wobei der Effektoranteil ein Interferon (IFN)-α2b ist; und
b) einen PEG-Anteil, der an eine Sequenz einer Ankerdomäne (AD) eines A-Kinase-Ankerproteins (AKAP) gebunden ist;
wobei zwei DDD-Sequenzen an eine AD-Sequenz binden, um einen PEGylierten Komplex auszubilden.

2. Komplex nach Anspruch 1, weiter umfassend Disulfidbindungen zwischen den DDD- und AD-Sequenzen.

3. Komplex nach Anspruch 1, wobei die DDD-Sequenz die 44 N-terminalen Aminosäuren von PKA RIIa umfasst.

4. Komplex nach Anspruch 1, wobei die DDD-Sequenz SEQ.ID.NO:2 umfasst.

5. Komplex nach Anspruch 1, wobei der PEG-Anteil an einem Ende mit einer MethoxyGruppe abgedeckt ist.

6. Komplex nach Anspruch 1, wobei der PEG-Anteil, der an eine AD-Sequenz gebunden ist, IMP350, IMP360, IMP362, IMP413, IMP457 umfasst,
wobei
IMP350 CGQIEYLAKQIVDNAIQQAGC(SS-tbu)-NH₂ (SEQ ID NO:1) ist,
IMP360 CGQIEYLAKQIVDNAIQQAGC(SS-tbu)G-EDANS (SEQ ID NO:1) ist,
IMP362 PEG₂₀- CGQIEYLAKQIVDNAIQQAGC(SS-tbu)G-EDANS ist,
IMP413 PEG₃₀- CGQIEYLAKQIVDNAIQQAGC(SS-tbu)G-EDANS ist, und
IMP457 das Reaktionsprodukt von Ac-C-PEG₃-C(S-*t*Bu)GQIEYLAKQIVDNAIQQAGC(S-*t*Bu)G-NH₂ und mPEG2-MAL-40K von Figur 16 ist, die wie in Beispiel 1 beschrieben umgesetzt sind.

7. Komplex nach Anspruch 1, wobei die Clearance-Geschwindigkeit des PEGylierten Komplexes aus Serum wenigstens eine Größenordnung langsamer ist als die Clearance-Geschwindigkeit des nicht-PEGylierten Effektoranteils.

8. PEGylierter Komplex umfassend:
a) einen Effektoranteil, der an eine Sequenz einer Ankerdomäne (AD) eines A-Kinase-Ankerproteins (AKAP) gebunden ist, wobei der Effektoranteil ein Interferon (IFN)-α2b ist; und
b) einen PEG-Anteil, der an eine Sequenz einer Dimerisierungs- und Kopplungsdomäne (DDD) von Proteinkinase A (PKA) RIIα gebunden ist;
wobei die zwei DDD-Sequenzen an eine AD-Sequenz binden, um einen PEGylierten Komplex auszubilden.

9. Komplex nach Anspruch 8, wobei die Clearance-Geschwindigkeit des PEGylierten Komplexes aus Serum wenigstens eine Größenordnung langsamer ist als die Clearance-Geschwindigkeit des nicht-PEGylierten Effektoranteils.

10. Verfahren zum PEGylieren eines Effektoranteils umfassend:
a) Binden eines Effektoranteils an eine Sequenz einer Dimerisierungs- und Kopplungsdomäne (DDD) von Proteinkinase A (PKA) RIIα, wobei der Effektoranteil ein Interferon (IFN)-α2b ist;
b) Binden eines PEG-Anteils an eine Sequenz einer Ankerdomäne (AD) von einem A-Kinase-Ankerprotein (AKAP); und
c) Erlauben, dass die DDD-Sequenz an die AD-Sequenz bindet, um einen PEGylierten Komplex auszubilden, der zwei Effektoranteil-DDD-Sequenzen und eine PEG-AD-Sequenz umfasst.

11. Verfahren nach Anspruch 10, wobei die Clearance-Geschwindigkeit des PEGylierten Komplexes aus Serum wenigstens eine Größenordnung langsamer ist als die Clearance-Geschwindigkeit des nicht-PEGylierten Effektoranteils.

12. Verfahren zum PEGylieren eines Effektoranteils umfassend:
a) Binden eines Effektoranteils an eine Sequenz einer Ankerdomäne (AD) eines A-Kinase-Ankerproteins (AKAP), wobei der Effektoranteil ein Interferon (IFN)-α2b ist;
b) Binden eines PEG-Anteils an eine Sequenz einer Dimerisierungs- und Kopplungsdomäne (DDD) von Proteinkinase A (PKA) RIIα; und
c) Erlauben, dass die DDD-Sequenz an die AD-Sequenz bindet, um einen PEGylierten Komplex auszubilden, der zwei Effektoranteil-DDD-Sequenzen und eine PEG-AD-Sequenz umfasst.

## Revendications

1. Complexe PEGylé comprenant :
a) un fragment effecteur lié à une séquence de domaine de dimérisation et d'ancrage (DDD) de protéine kinase A (PKA) RIIα, **caractérisé en ce que** le fragment effecteur est un interféron (IFN)-α2b ; et
b) un fragment PEG lié à une séquence de domaine d'ancrage (AD) d'une protéine d'ancrage de kinase A (AKAP) ;
dans lequel deux séquences DDD se lient à une séquence AD pour former un complexe PEGylé.

2. Complexe selon la revendication 1, comprenant en outre des ponts disulfure entre les séquences DDD et AD.

3. Complexe selon la revendication 1, dans lequel la séquence DDD comprend les 44 acides aminés N-terminaux de PKA RIIa.

4. Complexe selon la revendication 1, dans lequel la séquence DDD comprend SEQ ID NO: 2.

5. Complexe selon la revendication 1, dans lequel le fragment PEG est coiffé à une extrémité avec un groupe méthoxy.

6. Complexe selon la revendication 1, dans lequel le fragment PEG lié à une séquence AD comprend IMP350, IMP360, IMP362, IMP413 or IMP457, **caractérisé en ce que**
IMP350 est CGQIEYLAKQIVDNAIQQAGC(SS-tbu-NH₂) (SEQ ID NO: 1),
IMP360 est CGQIEYLAKQIVDNAIQQAGC(SS-tbu)G-EDANS (SEQ ID NO: 1),
IMP362 est PEG₂₀-CGQIEYLAKQIVDNAIQQAGC(SS-tbu)G-EDANS,
IMP413 est PEG₃₀-CGQIEYLAKQIVDNAIQQAGC(SS-tbu)G-EDANS, et
IMP457 est le produit de réaction de Ac-C-PEG₃-C(S-*t*Bu)GQIEYLAKQIVDNAIQQAGC(S-*t*Bu)G-NH₂ et mPEG2-MAL-40K de la figure 16 réagissant comme décrit dans l'Exemple 1.

7. Complexe selon la revendication 1, **caractérisé en ce que** le taux de clairance du complexe PEGylé depuis le sérum est d'au moins un ordre de grandeur plus lent que le taux de clairance du fragment effecteur non PEGylé.

8. Complexe PEGylé comprenant :
a) un fragment effecteur lié à une séquence de domaine d'ancrage (AD) d'une protéine d'ancrage de kinase A (AKAP), le fragment effecteur étant un interféron (IFN)-α2b ; et
b) un fragment PEG lié à une séquence de domaine de dimérisation et d'ancrage (DDD) de protéine kinase A (PKA) RIIα ;
**caractérisé en ce que** deux séquences DDD se lient à une séquence AD pour former un complexe PEGylé.

9. Complexe selon la revendication 8, **caractérisé en ce que** le taux de clairance du complexe PEGylé depuis le sérum est d'au moins un ordre de grandeur plus lent que le taux de clairance du fragment effecteur non PEGylé.

10. Procédé de PEGylation d'un fragment effecteur comprenant :
a) la liaison d'un fragment effecteur à une séquence de domaine de dimérisation et d'ancrage (DDD) de protéine kinase A (PKA) RIIα, **caractérisé en ce que** le fragment effecteur est un interféron (IFN)-α2b ;
b) la liaison d'un fragment PEG à une séquence de domaine d'ancrage (AD) d'une protéine d'ancrage de kinase A (AKAP) ; et
c) la conduite de la liaison de la séquence DDD à la séquence AD pour former un complexe PEGylé comprenant deux séquences fragment effecteur-DDD et une séquence PEG-AD.

11. Procédé selon la revendication 10, dans lequel le taux de clairance du complexe PEGylé depuis le sérum est au moins un ordre de grandeur plus lent que le taux de clairance du fragment effecteur non PEGylé.

12. Procédé de PEGylation d'un fragment effecteur comprenant :
a) la liaison d'un fragment effecteur à une séquence de domaine d'ancrage (AD) d'une protéine d'ancrage de kinase A (AKAP), **caractérisé en ce que** le fragment effecteur est un interféron (IFN)-α2b ;
b) la liaison d'un fragment PEG à une séquence de domaine de dimérisation et d'ancrage (DDD) de protéine kinase A (PKA) RIIα ; et
c) la conduite de la liaison de la séquence DDD à la séquence AD pour former un complexe PEGylé comprenant deux séquences fragment effecteur-DDD et une séquence PEG-AD.


### SEQUENCE LISTING

<110> IBC PHARMACEUTICALS, INC.
<120> PEGYLATION BY THE DOCK AND LOCK (DNL) TECHNIQUE
<130> I 10107 EP
<140> EP 08 842 661.4
   <141> 2008-10-24
<150> 11/925,408
   <151> 2007-10-26
<160> 33
<170> PatentIn version 3.5
<210> 1
   <211> 21
   <212> PRT
   <213> Artificial Sequence
<220>
   <223> Description of Artificial Sequence: Synthetic peptide
<400> 1
   Cys Gly Gln Ile Glu Tyr Leu Ala Lys Gln Ile Val Asp Asn Ala Ile
   1               5                   10                  15
   Gln Gln Ala Gly Cys
               20
<210> 2
   <211> 63
   <212> PRT
   <213> Artificial Sequence
<220>
   <223> Description of Artificial Sequence: Synthetic peptide
<400> 2
   Lys Ser His His His His His His Gly Ser Gly Gly Gly Gly Ser Gly
   1               5                   10                  15
   Gly Gly Cys Gly His Ile Gln Ile Pro Pro Gly Leu Thr Glu Leu Leu
               20                  25                  30
   Gln Gly Tyr Thr Val Glu Val Leu Arg Gln Gln Pro Pro Asp Leu Val
           35                  40                  45
   Glu Phe Ala Val Glu Tyr Phe Thr Arg Leu Arg Glu Ala Arg Ala
       50                  55                  60
<210> 3
   <211> 45
   <212> DNA
   <213> Artificial Sequence
<220>
   <223> Description of Artificial Sequence: Synthetic oligonucleotide
<400> 3
   tctagacaca ggacctcatc atggccttga cctttgcttt actgg 45
<210> 4
   <211> 55
   <212> DNA
   <213> Artificial Sequence
<220>
   <223> Description of Artificial Sequence: Synthetic oligonucleotide
<400> 4
   ggatccatga tggtgatgat ggtgtgactt ttccttactt cttaaacttt cttgc 55
<210> 5
   <211> 41
   <212> DNA
   <213> Artificial Sequence
<220>
   <223> Description of Artificial Sequence: Synthetic oligonucleotide
<400> 5
   tctagacaca ggacctcatc atggctggac ctgccaccca g 41
<210> 6
   <211> 51
   <212> DNA
   <213> Artificial Sequence
<220>
   <223> Description of Artificial Sequence: Synthetic oligonucleotide
<400> 6
   ggatccatga tggtgatgat ggtgtgactt gggctgggca aggtggcgta g 51
<210> 7
   <211> 40
   <212> DNA
   <213> Artificial Sequence
<220>
   <223> Description of Artificial Sequence: Synthetic oligonucleotide
<400> 7
   tctagacaca ggacctcatc atgggggtgc acgaatgtcc 40
<210> 8
   <211> 49
   <212> DNA
   <213> Artificial Sequence
<220>
   <223> Description of Artificial Sequence: Synthetic oligonucleotide
<400> 8
   ggatccatga tggtgatgat ggtgtgactt tctgtcccct gtcctgcag 49
<210> 9
   <211> 22
   <212> PRT
   <213> Artificial Sequence
<220>
   <223> Description of Artificial Sequence: Synthetic peptide
<220>
   <221> MOD_RES
   <222> (1)..(1)
   <223> Cys(S-tBu)
<220>
   <221> MOD_RES
   <222> (21)..(21)
   <223> Cys(S-tBu)
<400> 9
   Cys Gly Gln Ile Glu Tyr Leu Ala Lys Gln Ile Val Asp Asn Ala Ile
   1               5                   10                  15
   Gln Gln Ala Gly Cys Gly
               20
<210> 10
   <211> 44
   <212> PRT
   <213> Artificial Sequence
<220>
   <223> Description of Artificial Sequence: Synthetic peptide
<400> 10
   Ser His Ile Gln Ile Pro Pro Gly Leu Thr Glu Leu Leu Gln Gly Tyr
   1               5                   10                  15
   Thr Val Glu Val Leu Arg Gln Gln Pro Pro Asp Leu Val Glu Phe Ala
               20                  25                  30
   Val Glu Tyr Phe Thr Arg Leu Arg Glu Ala Arg Ala
           35                  40
<210> 11
   <211> 45
   <212> PRT
   <213> Artificial Sequence
<220>
   <223> Description of Artificial Sequence: Synthetic peptide
<400> 11
   Cys Gly His Ile Gln Ile Pro Pro Gly Leu Thr Glu Leu Leu Gln Gly
   1               5                   10                  15
   Tyr Thr Val Glu Val Leu Arg Gln Gln Pro Pro Asp Leu Val Glu Phe
               20                  25                  30
   Ala Val Glu Tyr Phe Thr Arg Leu Arg Glu Ala Arg Ala
           35                  40                  45
<210> 12
   <211> 17
   <212> PRT
   <213> Artificial Sequence
<220>
   <223> Description of Artificial Sequence: Synthetic peptide
<400> 12
   Gln Ile Glu Tyr Leu Ala Lys Gln Ile Val Asp Asn Ala Ile Gln Gln
   1               5                   10                  15
   Ala
<210> 13
   <211> 20
   <212> DNA
   <213> Artificial Sequence
<220>
   <223> Description of Artificial Sequence: Synthetic oligonucleotide
<400> 13
   gaacctcgcg gacagttaag 20
<210> 14
   <211> 53
   <212> DNA
   <213> Artificial Sequence
<220>
   <223> Description of Artificial Sequence: Synthetic oligonucleotide
<400> 14
   ggatcctccg ccgccgcagc tcttaggttt cttgtccacc ttggtgttgc tgg 53
<210> 15
   <211> 55
   <212> PRT
   <213> Artificial Sequence
<220>
   <223> Description of Artificial Sequence: Synthetic peptide
<400> 15
   Gly Ser Gly Gly Gly Gly Ser Gly Gly Gly Gly Ser His Ile Gln Ile
   1               5                   10                  15
   Pro Pro Gly Leu Thr Glu Leu Leu Gln Gly Tyr Thr Val Glu Val Leu
               20                  25                  30
   Arg Gln Gln Pro Pro Asp Leu Val Glu Phe Ala Val Glu Tyr Phe Thr
           35                  40                  45
   Arg Leu Arg Glu Ala Arg Ala
       50                  55
<210> 16
   <211> 92
   <212> DNA
   <213> Artificial Sequence
<220>
   <223> Description of Artificial Sequence: Synthetic oligonucleotide
<400> 16
   gtggcgggtc tggcggaggt ggcagccaca tccagatccc gccggggctc acggagctgc     60
   tgcagggcta cacggtggag gtgctgcgac ag                                   92
<210> 17
   <211> 92
   <212> DNA
   <213> Artificial Sequence
<220>
   <223> Description of Artificial Sequence: Synthetic oligonucleotide
<400> 17
   gcgcgagctt ctctcaggcg ggtgaagtac tccactgcga attcgacgag gtcaggcggc     60
   tgctgtcgca gcacctccac cgtgtagccc tg                                   92
<210> 18
   <211> 30
   <212> DNA
   <213> Artificial Sequence
<220>
   <223> Description of Artificial Sequence: Synthetic oligonucleotide
<400> 18
   ggatccggag gtggcgggtc tggcggaggt 30
<210> 19
   <211> 30
   <212> DNA
   <213> Artificial Sequence
<220>
   <223> Description of Artificial Sequence: Synthetic oligonucleotide
<400> 19
   cggccgtcaa gcgcgagctt ctctcaggcg 30
<210> 20
   <211> 29
   <212> PRT
   <213> Artificial Sequence
<220>
   <223> Description of Artificial Sequence: Synthetic peptide
<400> 20
   Gly Ser Gly Gly Gly Gly Ser Gly Gly Gly Gly Ser Gln Ile Glu Tyr
   1               5                   10                  15
   Leu Ala Lys Gln Ile Val Asp Asn Ala Ile Gln Gln Ala
               20                  25
<210> 21
   <211> 96
   <212> DNA
   <213> Artificial Sequence
<220>
   <223> Description of Artificial Sequence: Synthetic oligonucleotide
<400> 21
   ggatccggag gtggcgggtc tggcggaggt ggcagccaga tcgagtacct ggccaagcag     60
   atcgtggaca acgccatcca gcaggcctga cggccg                               96
<210> 22
   <211> 96
   <212> DNA
   <213> Artificial Sequence
<220>
   <223> Description of Artificial Sequence: Synthetic oligonucleotide
<400> 22
   cggccgtcag gcctgctgga tggcgttgtc cacgatctgc ttggccaggt actcgatctg 60
   gctgccacct ccgccagacc cgccacctcc ggatcc 96
<210> 23
   <211> 30
   <212> DNA
   <213> Artificial Sequence
<220>
   <223> Description of Artificial Sequence: Synthetic oligonucleotide
<400> 23
   ggatccggag gtggcgggtc tggcggaggt 30
<210> 24
   <211> 22
   <212> DNA
   <213> Artificial Sequence
<220>
   <223> Description of Artificial Sequence: Synthetic oligonucleotide
<400> 24
   cggccgtcag gcctgctgga tg 22
<210> 25
   <211> 10
   <212> PRT
   <213> Artificial Sequence
<220>
   <223> Description of Artificial Sequence: Synthetic peptide
<400> 25
   Gly Gly Gly Gly Ser Gly Gly Gly Cys Gly
   1 5 10
<210> 26
   <211> 73
   <212> DNA
   <213> Artificial Sequence
<220>
   <223> Description of Artificial Sequence: Synthetic oligonucleotide
<400> 26
   gatccggagg tggcgggtct ggcggaggtt gcggccacat ccagatcccg ccggggctca 60
   cggagctgct gca 73
<210> 27
   <211> 65
   <212> DNA
   <213> Artificial Sequence
<220>
   <223> Description of Artificial Sequence: Synthetic oligonucleotide
<400> 27
   gcagctccgt gagccccggc gggatctgga tgtggccgca acctccgcca gacccgccac 60
   ctccg 65
<210> 28
   <211> 24
   <212> DNA
   <213> Artificial Sequence
<220>
   <223> Description of Artificial Sequence: Synthetic oligonucleotide
<400> 28
   agatctggcg cacctgaact cctg 24
<210> 29
   <211> 33
   <212> DNA
   <213> Artificial Sequence
<220>
   <223> Description of Artificial Sequence: Synthetic oligonucleotide
<400> 29
   gaattcggat cctttacccg gagacaggga gag 33
<210> 30
   <211> 21
   <212> PRT
   <213> Artificial Sequence
<220>
   <223> Description of Artificial Sequence: Synthetic peptide
<220>
   <221> MOD_RES
   <222> (21)..(21)
   <223> Cys(SS-tBu)
<400> 30
   Cys Gly Gln Ile Glu Tyr Leu Ala Lys Gln Ile Val Asp Asn Ala Ile
   1               5                   10                  15
   Gln Gln Ala Gly Cys
               20
<210> 31
   <211> 22
   <212> PRT
   <213> Artificial Sequence
<220>
   <223> Description of Artificial Sequence: Synthetic peptide
<220>
   <221> MOD_RES
   <222> (21)..(21)
   <223> Cys(SS-tBu)
<220>
   <221> MOD_RES
   <222> (22)..(22)
   <223> Gly-EDANS
<400> 31
   Cys Gly Gln Ile Glu Tyr Leu Ala Lys Gln Ile Val Asp Asn Ala Ile
   1               5                   10                  15
   Gln Gln Ala Gly Cys Gly
               20
<210> 32
   <211> 6
   <212> PRT
   <213> Artificial Sequence
<220>
   <223> Description of Artificial Sequence: Synthetic 6xHis tag
<400> 32
   His His His His His His
   1               5
<210> 33
   <211> 10
   <212> PRT
   <213> Artificial Sequence
<220>
   <223> Description of Artificial Sequence: Synthetic peptide
<400> 33
   Gly Gly Gly Gly Ser Gly Gly Gly Gly Ser
   1               5                   10